(19) Europäisches Patentamt
European Patent Office
Office européen des brevets

(11) **EP 4 786 605 A1**

(12) **EUROPEAN PATENT APPLICATION**
published in accordance with Art. 153(4) EPC

(43) Date of publication:
**05.08.2026 Bulletin 2026/32**

(21) Application number: **24873000.4**

(22) Date of filing: **26.09.2024**

(51) International Patent Classification (IPC):
**C12Q 1/48** (2006.01)    **C12Q 1/686** (2018.01)

(52) Cooperative Patent Classification (CPC):
**C12Q 1/48; C12Q 1/686**

(86) International application number:
**PCT/KR2024/014643**

(87) International publication number:
**WO 2025/071292 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.09.2023 KR 20230129456**

(71) Applicant: **Seegene, Inc.**
**Seoul 05548 (KR)**

(72) Inventors:
• **AHN, Byungjun**
**Hanam-si, Gyeonggi-do 12955 (KR)**
• **JUNG, Youjin**
**Seoul 08725 (KR)**
• **LEE, Jaemin**
**Yongin-si, Gyeonggi-do 16832 (KR)**
• **KIM, Myung-Il**
**Hanam-si, Gyeonggi-do 12916 (KR)**

(74) Representative: **Kilger, Ute**
**Boehmert & Boehmert**
**Anwaltspartnerschaft mbB**
**Pettenkoferstrasse 22**
**80336 München (DE)**

(54) **METHOD FOR EVALUATING PERFORMANCE OF HOT START NUCLEIC ACID POLYMERASE**

(57)    The present invention relates to a method for evaluating the performance of a hot start nucleic acid polymerase, a method for obtaining a quality controlcapable (QC-able) hot start nucleic acid polymerase, and a method for providing a blended hot start nucleic acid polymerase that mimics the performance of a target hot-start nucleic acid polymerase. According to the present invention, by using an activity profile of a hot start nucleic acid polymerase for heat activation time, it is possible to evaluate the performance of the hot start nucleic acid polymerase, to provide a QC-able hot start nucleic acid polymerase, and to provide a blended nucleic acid polymerase that mimics the performance of a target hot start nucleic acid polymerase.

**FIG. 1**

*100*

110 — obtaining a product containing a hot start nucleic acid polymerase

120 — aliquoting the product containing a hot start nucleic acid polymerase and performing heat activation on each of the aliquoted products for different times

130 — measuring an activity of each hot start nucleic acid polymerase contained in each of the heat-activated products for different times

140 — obtaining the activity of the hot start nucleic acid polymerase for the different heat activation times as an activity profile of the hot start nucleic acid polymerase

EP 4 786 605 A1

**Description**

**CROSS-REFERENCE TO RELATED APPLICATIONS**

This patent application claims priority to Korean Patent Application No. 2023-0129456 filed with the Korean Intellectual Property Office on September 26, 2023, and the disclosure of the patent applications is inserted into this specification by reference.

**FIELD OF THE INVENTION**

The present invention relates to a method for evaluating a performance of a hot start nucleic acid polymerase, a method for obtaining a QC-able hot start nucleic acid polymerase, or a method for providing blended hot start nucleic acid polymerases that mimic a performance of a target hot start nucleic acid polymerase.

**DESCRIPTION OF THE RELATED ART**

Molecular diagnostics is one of in *vitro* diagnostic testing methods, and generally includes a method of amplifying DNA or RNA using polymerase chain reaction (PCR) and then performing detection or analysis. PCR continues to be important, even in molecular diagnostic testing for proactive responses to infectious diseases such as COVID-19. To enable such PCR, it is highly important to utilize enzymes that are stable across various temperatures used in the reaction and that do not lose activity. At present, most molecular diagnostic reagents based on PCR utilize polymerases derived from microorganisms that are stable at high temperatures (extreme thermophiles), and a representative example is *Taq* DNA polymerase derived from *T. aquaticus* (Chien A., Edgar D. B., Trela J. M., J. Bacteriol., 1976, 127, 1550-1557).

Among polymerases used for molecular diagnostics, DNA polymerases are enzymes that catalyze polymerization of DNA using DNA or RNA as a template. In general, polymerases consume nucleoside triphosphates as substrates and because their primary function is polymerization, enzyme activity may be assessed based on how rapidly the substrate is consumed and incorporated into a polymer. Such activity is defined as a characteristic property of the enzyme, namely an activity unit (Unit).

An activity unit of a polymerase is defined as the amount of enzyme that causes, at a defined temperature and within a defined time period, a defined amount of nucleoside triphosphates to be polymerized into an acid-insoluble form. For example, New England Biolabs defines 1 Unit of Taq DNA polymerase as the amount of enzyme that converts 15 nmol of nucleoside triphosphates into an acid-insoluble form in 30 minutes at 75°C. Similarly, for Bst DNA polymerase (large fragment), 1 unit is defined as the amount of enzyme that converts 10 nmol of nucleoside triphosphates into an acid-insoluble form in 30 minutes at 65°C.

Although the definition of a unit (Unit) may vary slightly among companies or products in terms of temperature, substrate amount, and types of materials including radioisotopes, the common feature is that the amount of consumed substrate or the amount of acid-insoluble polymer is measured using radioisotopes and the reaction rate is calculated to define the unit (Charles C. Richardson, Carl L. Schildkraut, H. Vasken Aposhian, Arthur Kornberg, J. Biol. Chem., 1964, 239(1), 222-232).

The definition of units serves not only as a performance indicator of molecular diagnostic reagents but may also function as an analytical method for diagnostics itself. Therefore, accurate analysis of polymerase unit measurement has become an important issue for various reasons.

Among methods for measuring enzyme units, radioisotope-based methods provide high accuracy, however, radioisotopes may cause environmental concerns and may adversely affect the health of experimenters. Accordingly, studies have been conducted for decades to define polymerase units without using radioisotopes. For example, attempts have been made to measure units based on changes in fluorescence of single-stranded DNA-binding proteins (Mark A. Griep, Anal. Biochem., 1995, 232, 180-189), to measure units by microplate reading of fluorescently labeled nucleotides, or to measure units using molecular beacons or electrochemical methods.

Because molecular diagnostic products play an important role in determining a patient's condition, sensitivity and specificity are critical. Such products must be capable of reliably diagnosing whether a virus or microorganism is present, or confirming that an infection is absent. Accordingly, prior to conducting an actual PCR reaction, it is necessary to prevent unintended diagnostic outcomes caused by enzyme errors or the like. PCR may yield inaccurate results due to several issues, for example, primers used in PCR may anneal to unintended positions of a template DNA, or primers may bind to each other non-specifically. If a polymerase exhibits residual activity at low temperatures and recognizes such non-specific annealing, unintended amplification may occur and undesired results may be produced. To prevent such unintended amplification reactions, hot start polymerases have been developed in which polymerase activity is inhibited before a high-temperature treatment.

Hot start methods may be categorized into physical methods, chemical methods, and other methods. Physical methods refer to suppressing reactions by physically separating the enzyme from reactants using wax, beads, or the like, until such separation is removed at or above a certain temperature. Chemical hot start methods refer to chemically modifying the enzyme itself to control the enzyme so that it exhibits no activity without a pretreatment at a certain temperature, and formaldehyde or citraconic anhydride has been used as representative reagents (U.S. Pat. No. 5,677,152). In addition, methods have been developed and reported in which reactions are suppressed using antibodies or aptamers that can bind to the polymerase, or in which essential magnesium ions or primers are controlled.

In most hot start methods, prior to performing the main PCR reaction, a heat pretreatment is applied to remove or weaken an inhibitor so that the enzyme participates only in the main reaction.

For use in diagnostic reagents, enzymes produced through manufacturing are added to products based on a specified activity unit according to product characteristics. However, as described above, because a hot start enzyme is generally in an inhibited state, conventional methods for measuring polymerase activity for diagnostic reagents may result in very low measured activity or no measurable activity. Therefore, it has been important to develop techniques for determining, through separate methods, whether the performance of such enzymes satisfies a certain level or whether a certain amount of enzyme is incorporated into a diagnostic reagent.

Enzymes used in diagnostic reagents undergo culturing and purification processes, and then typically undergo additional, extended reaction processes for imparting hot start performance, making it difficult to consistently produce hot start enzymes with the same quality. However, if the quality of a final hot start enzyme product can be precisely analyzed and quantified, the quality of enzymes used to manufacture consistent diagnostic reagent products can be improved despite such challenges.

Accordingly, the inventors recognized a need to develop a method capable of monitoring enzyme activity and quality by providing a series of methods for inspecting the final performance and quality of hot start enzymes for diagnostics.

Throughout this application, various patents and publications are referenced and citations are provided in parentheses. The disclosure of these patents and publications in their entities are hereby incorporated by references into this application in order to more fully describe this invention and the state of the art to which this invention pertains.

## SUMMARY OF THE INVENTION

The inventors have endeavored to develop a method for analyzing the performance of a hot start nucleic acid polymerase capable of monitoring the activity and quality of the hot start nucleic acid polymerase. As a result, the inventors confirmed that, by establishing an activity profile of a hot start nucleic acid polymerase with respect to heat activation time using a method for measuring activity (unit) of a nucleic acid polymerase without using radioisotopes, it is possible to evaluate the performance of the hot start nucleic acid polymerase, to provide a QC-able hot start nucleic acid polymerase, and to provide blended nucleic acid polymerases that mimic the performance of a target hot start nucleic acid polymerase. Accordingly, the present invention has been completed.

Accordingly, it is an object of the present invention to provide a method for evaluating a performance of a hot start nucleic acid polymerase.

It is another object of the present invention to provide a method for obtaining a QC-able hot start nucleic acid polymerase.

It is still another object of the present invention to provide a method for providing blended hot start nucleic acid polymerases that mimic a performance of a target hot start nucleic acid polymerase.

Other objects and advantages of the present invention will become apparent from the detailed description to follow taken in conjugation with the appended claims and drawings.

## DETAILED DESCRIPTION OF THIS INVETNION

### I. Method for Evaluating Performance of a Hot Start Nucleic Acid Polymerase (First Aspect)

In an aspect of the present invention, there is provided a method for evaluating a performance of a hot start nucleic acid polymerase, comprising the following steps:

(a) obtaining a product containing a hot start nucleic acid polymerase;
(b) aliquoting the product containing the hot start nucleic acid polymerase and performing heat activation on each of the aliquoted products for different times;
(c) measuring an activity of each hot start nucleic acid polymerase contained in each of the heat-activated products for different times; wherein the activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization condition to form an extended duplex, and a detectable signal is provided in a manner dependent on the extended duplex; and

(d) obtaining the activity of the hot start nucleic acid polymerase for the different heat activation times as an activity profile of the hot start nucleic acid polymerase; wherein the activity profile of the hot start nucleic acid polymerase indicates the performance of the hot start nucleic acid polymerase.

According to the method of the present invention, by constructing an activity profile of a hot start nucleic acid polymerase with respect to heat activation time using a method for measuring activity (unit) of a nucleic acid polymerase without using radioisotopes, the performance of the hot start nucleic acid polymerase can be evaluated.

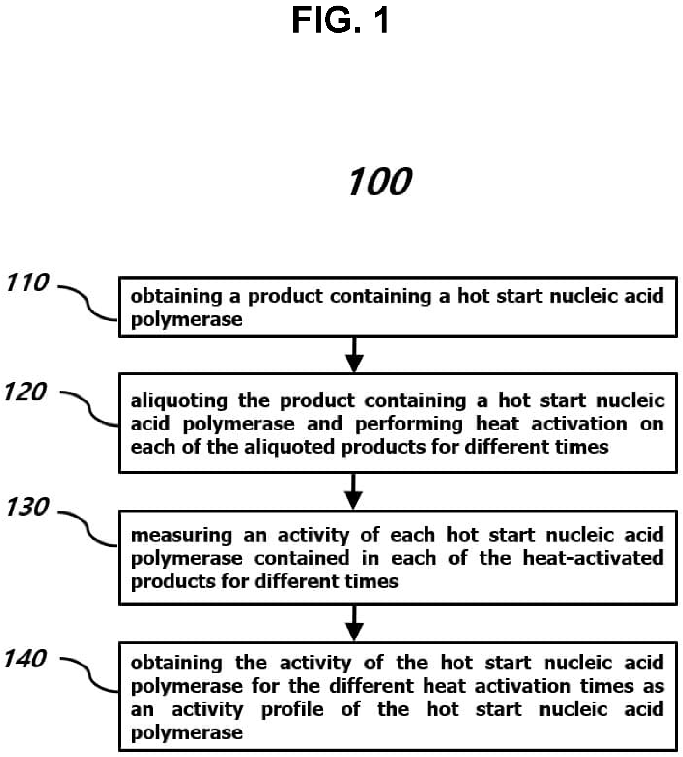

FIG. 1 is a flowchart illustrating processes for performing the method of the present invention according to one embodiment. The method of the present invention will be described below with reference to FIG. 1.

Step (a): obtaining a product containing a hot start nucleic acid polymerase (**110**)

First, the method of the present invention comprises step (a) of obtaining a product containing a hot start nucleic acid polymerase.

As used herein, the term "hot start nucleic acid polymerase" refers to a nucleic acid polymerase whose activity is suppressed at room temperature or low temperature through chemical modification or additives prior to exposure to a high temperature, in order to prevent unintended amplification reactions.

According to one embodiment of the present invention, the hot start nucleic acid polymerase is a chemically modified hot start nucleic acid polymerase, an antibody-based hot start nucleic acid polymerase, or an aptamer-based hot start nucleic acid polymerase. Most specifically, the hot start nucleic acid polymerase is a chemically modified hot start nucleic acid polymerase.

Chemical reagents used to produce the chemically modified hot start nucleic acid polymerase may include various chemical substances known in the art, and examples thereof include formaldehyde and citraconic anhydride.

As used herein, the nucleic acid polymerase is an enzyme that polymerizes DNA using DNA or RNA as a template, and is specifically a DNA polymerase, and more specifically *Taq* DNA polymerase, KlenTaq DNA polymerase, a Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase, or HIV reverse transcriptase.

According to one embodiment, the nucleic acid polymerase is prepared by a method comprising the following steps: (a-1) culturing host cells containing an expression construct for a nucleic acid polymerase; and (a-2) purifying the nucleic acid polymerase by applying the resultant of step (a-1) to column chromatography.

The preparation process of the nucleic acid polymerase generally comprises (a-1) a culturing process and (a-2) a purification process.

(a-1) Culturing Process

As used herein, the term "expression construct for a nucleic acid polymerase" refers to a nucleic acid molecule comprising only minimal elements required for expression of a nucleic acid polymerase in a cell.

According to one embodiment of the present invention, the expression construct for a nucleic acid polymerase is a vector comprising (i) a nucleotide sequence encoding a nucleic acid polymerase, and (ii) a promoter.

As used herein, the term "nucleotide" refers to a deoxyribonucleotide or ribonucleotide present in a single-stranded or double-stranded form, and includes analogs of naturally occurring nucleotides unless otherwise specified (Scheit, Nucleotide Analogs, John Wiley, New York (1980); Uhlmann and Peyman, Chemical Reviews, 90:543-584 (1990)).

As used herein, the term "promoter" refers to an untranslated nucleic acid sequence upstream of a coding region, which comprises a binding site for a polymerase and has transcription initiation activity for a downstream gene of the promoter into mRNA.

The expression construct may be constructed using various methods known in the art, and detailed methods are disclosed in Sambrook et al., Molecular Cloning: A Laboratory Manual, Cold Spring Harbor Laboratory Press (2001), which is incorporated herein by reference.

When the expression construct is typically constructed as an expression vector, the vector may be constructed using a prokaryotic cell or a eukaryotic cell as a host cell, and more specifically using a prokaryotic cell as a host cell. When the vector is an expression vector using a prokaryotic host cell, the vector generally comprises a promoter having strong transcriptional activity (for example, a *tac* promoter, *lac* promoter, lacUV5 promoter, *lpp* promoter, pLλ promoter, pRλ promoter, rac5 promoter, *amp* promoter, recA promoter, SP6 promoter, *trp* promoter, T7 promoter, or *phoB* promoter), a ribosome binding site for initiation of translation, and transcription/translation termination sequences.

When *E. coli* (e.g., HB101, BL21, DH5α, etc.) is used as the host cell, a promoter and operator region of the *E. coli* tryptophan biosynthetic pathway (Yanofsky, C., J. Bacteriol., 158:1018-1024 (1984)) and a leftward promoter of bacteriophage λ (pL λ promoter; Herskowitz, I. and Hagen, D., Annu. Rev. Genet., 14:399-445 (1980)) may be used as the promoter.

When a *Bacillus* strain is used as the host cell, a promoter of a toxin protein gene of Bacillus *thuringiensis* (Appl. Environ. Microbiol., 64:3932-3938 (1998); Mol. Gen. Genet., 250:734-741 (1996)) or any promoter that is expressible in a *Bacillus* strain may be used as the promoter.

When the vector is prepared for expression in a eukaryotic cell, available promoters include, but are not limited to, a CMV promoter, an adenovirus late promoter, a vaccinia virus 7.5K promoter, an SV40 promoter, an HSV *tk* promoter, an RSV promoter, an EF1α promoter, a metallothionein promoter, a β-actin promoter, a human IL-2 gene promoter, a human IFN gene promoter, a human IL-4 gene promoter, a human lymphotoxin gene promoter, and a human GM-CSF gene promoter.

For a vector for expression in a eukaryotic cell, it is preferable that a polyadenylation sequence is operably linked downstream of the nucleotide sequence encoding the nucleic acid polymerase. Examples of such polyadenylation sequences include, but are not limited to, an SV40-derived polyadenylation sequence (Schek, N. et al., Mol. Cell Biol., 12:5386-5393 (1992)), HIV-1 polyA (Klasens, B. I. F. et al., Nucleic Acids Res., 26:1870-1876 (1998)), β-globin polyA (Gil, A. et al., Cell, 49:399-406 (1987)), HSV TK polyA (Cole, C. N. and Stacy, T. P., Mol. Cell Biol., 5:2104-2113 (1985)), and polyomavirus polyA (Batt, D. B. and Carmichael, G. G., Mol. Cell Biol., 15:4783-4790 (1995)).

As used herein, the term "nucleic acid polymerase-coding nucleotide" refers to a nucleotide corresponding to a coding sequence (CDS) of a nucleic acid polymerase, which is a target protein.

The expression construct may further comprise a selectable marker for selection. Specifically, the selectable marker is a polynucleotide encoding a gene conferring antibiotic resistance. Examples of antibiotic resistance genes include resistance genes against ampicillin, gentamicin, carbenicillin, chloramphenicol, streptomycin, kanamycin, geneticin, neomycin, and tetracycline.

More specifically, the vector may be produced by manipulating a plasmid (*e.g.*, pDG1661, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), a phage (*e.g.*, λgt4·λB, λ-Charon, λΔz1, and M13), or a virus (*e.g.*, SV40), and most specifically is produced by manipulating a plasmid.

Meanwhile, methods for introducing the above-described vector into a cell may be performed using various methods known in the art. For example, the vector may be introduced into a cell by microinjection (Capecchi, M. R., Cell, 22:479 (1980); Harland and Weintraub, J. Cell Biol., 101:1094-1099 (1985)), calcium phosphate precipitation (Graham, F. L. et al., Virology, 52:456 (1973); Chen and Okayama, Mol. Cell Biol., 7:2745-2752 (1987)), electroporation (Neumann, E. et al., EMBO J., 1:841 (1982); Tur-Kaspa et al., Mol. Cell Biol., 6:716-718 (1986)), liposome-mediated transfection (Wong, T. K. et al., Gene, 10:87 (1980); Nicolau and Sene, Biochim. Biophys. Acta, 721:185-190 (1982); Nicolau et al., Methods Enzymol., 149:157-176 (1987)), DEAE-dextran treatment (Gopal, Mol. Cell Biol., 5:1188-1190 (1985)), or gene bombardment (Yang et al., Proc. Natl. Acad. Sci., 87:9568-9572 (1990)).

Any host cell known in the art that is capable of stably and continuously expressing the expression construct may be used as the host cell. The host cell may be a prokaryotic cell or a eukaryotic cell.

When a prokaryotic cell is used as the host cell, examples thereof include *E. coli* JM109, *E. coli* BL21, *E. coli* RR1, *E. coli* LE392, *E. coli* B, *E. coli* X1776, *E. coli* W3110, Bacillus *subtilis*, Bacillus *thuringiensis*, and enteric bacterial strains such as Salmonella *typhimurium,* Serratia *marcescens*, and various Pseudomonas species. When a eukaryotic cell is used as the host cell, examples thereof include yeast (Saccharomyces cerevisiae), insect cells, and human cells (*e.g.*, CHO cells (Chinese hamster ovary), W138, BHK, COS-7, 293, HepG2, 3T3, RIN, and MDCK cell lines).

According to one embodiment of the present invention, the host cell is a prokaryotic cell, and most specifically *E. coli*.

Culturing of the transformed host cells may be performed using media commonly used in the art. For example, when the transformed host cells are prokaryotic cells (*e.g.*, *E. coli*), culturing may be performed using LB (Luria-Bertani) medium. Various culturing methods for transformed host cells are well known to those skilled in the art and are disclosed in Sambrook et al., Molecular Cloning, A Laboratory Manual, Cold Spring Harbor Laboratory Press (2001), which is incorporated herein by reference.

According to a more specific embodiment, step (a-1) further comprises (a-1-1) disrupting the cultured host cells.

The cultured host cells may be disrupted by various methods known in the art. Specifically, disruption may be performed by sonication or by using glass beads, and more specifically, disruption of the cultured host cells is performed by sonication.

According to a more specific embodiment of the present invention, step (a-1) further comprises (a-1-2) heat-treating the cultured host cells or a lysate obtained by disrupting the cultured host cells. More specifically, the heat treatment is performed at 60°C to 120°C for 30 to 120 minutes.

(a-2) Purification Process

As used herein, the term "chromatography" refers to a process of separating a solute of interest in a mixture from other solutes in the mixture based on differences in migration rates of each solute through a stationary phase under the influence of a mobile phase.

The purification process may be performed using protein purification methods known in the art. For example, purification may be performed by filtration using a silica gel or celite gel column, size-exclusion chromatography using liquid column chromatography, ion-exchange chromatography, partition chromatography, affinity chromatography, or a combination thereof.

According to one embodiment, step (a-2) comprises (a-2-1) purifying the nucleic acid polymerase by applying the resultant of the step to ion-exchange chromatography; and (a-2-2) purifying the nucleic acid polymerase by applying the resultant of the step to affinity chromatography.

Specifically, step (a-2-1) is performed by anion-exchange column chromatography, and more specifically by quaternary amine-functionalized anion-exchange column chromatography, using the resultant of the step.

Specifically, for purification by affinity chromatography, the nucleic acid polymerase used in the present invention is in the form of a fusion protein fused with one selected from the group consisting of glutathione-S-transferase (GST), maltose binding protein (MBP), FLAG, 6× His (hexahistidine), NusA (N utilization substance A), and Trx (thioredoxin). For example, when glutathione-S-transferase is fused to the nucleic acid polymerase, glutathione, which is a substrate of the enzyme, may be used, and when a 6× His (His-tag) is used, purification may be performed rapidly and easily using a column packed with Ni-NTA resin.

According to one embodiment of the present invention, the hot start nucleic acid polymerase of step (a) is a nucleic acid polymerase with inhibited polymerization activity, and more specifically, the hot start nucleic acid polymerase is a nucleic acid polymerase whose polymerization activity is suppressed to 1 U/$\mu$g or less.

According to one embodiment, the method further comprises, after step (a), (a-3) diluting the product(s) to a concentration of 0.02 mg/mL to 2 mg/mL. More specifically, the product(s) are diluted to a concentration of 0.03 mg/mL to 2 mg/mL, and even more specifically to 0.05 mg/mL to 0.8 mg/mL. When the concentration of the product exceeds 2 mg/mL, there is a problem in that a real-time fluorescence value reaches a maximum too rapidly, making it difficult to obtain an analytical value suitable for differentiation. On the other hand, when the concentration is less than 0.02 mg/mL, fluorescence measurement based on double-stranded DNA binding does not occur properly, resulting in difficulty in measuring enzyme activity.

The dilution of step (a-3) may be performed using a reaction buffer.

Measurement of the concentration of the hot start nucleic acid polymerase contained in the product may be performed using various methods known in the art. For example, the concentration of the hot start nucleic acid polymerase may be measured using a Bradford assay, a BCA assay, or A280 absorbance.

Step (b): <u>heat activation for different times for each of the aliquoted products (**120**)</u>

Next, the method of the present invention comprises step (b) of aliquoting the product containing a hot start nucleic acid polymerase and performing heat activation on each of the aliquoted products for different times.

As used herein, the term "heat activation" refers to reversibly restoring activity of a hot start nucleic acid polymerase whose activity has been suppressed by physical, chemical, or other methods, by applying heat thereto.

According to one embodiment of the present invention, the heat activation of step (b) is performed on each of the aliquoted products for at least two time intervals selected from 0 minutes to 60 minutes. For example, heat activation may be performed for 1) 0 minutes and 60 minutes; 2) 15 minutes and 30 minutes; 3) 0 minutes, 15 minutes, and 60 minutes; 4) 0 minutes, 15 minutes, and 30 minutes; 5) 0 minutes, 15 minutes, 30 minutes, and 60 minutes; or 6) 0 minutes, 15 minutes, 30 minutes, 45 minutes, and 60 minutes.

More specifically, the heat activation of step (b) is performed on each of the aliquoted products for at least two time intervals selected from 0 minutes, 15 minutes, 30 minutes, 45 minutes, and 60 minutes. More specifically, the at least two time intervals may be 1) 0 minutes and 15 minutes; 2) 0 minutes, 15 minutes, and 30 minutes; 3) 0 minutes, 15 minutes, and 60 minutes; 4) 0 minutes, 15 minutes, 30 minutes, and 45 minutes; 5) 0 minutes, 15 minutes, 30 minutes, and 60 minutes; or 6) 0 minutes, 15 minutes, 30 minutes, 45 minutes, and 60 minutes.

According to one embodiment, the heat activation of step (b) is performed at 90°C to 100°C. More specifically, the heat activation is performed at 92°C to 98°C, and even more specifically at 94°C to 96°C.

According to one embodiment of the present invention, the heat activation of step (b) is performed on each of the aliquoted products at 90°C to 100°C for 0 minutes, 15 minutes, 30 minutes, and 60 minutes.

According to one embodiment, the method further comprises, between steps (b) and (c), (b-1) diluting each of the heat-activated products to a concentration of 6.25 ng/$\mu$L to 50 ng/$\mu$L. More specifically, each of the heat-activated products is diluted to a concentration of 6.25 ng/$\mu$L to 12.5 ng/$\mu$L. When the concentration is within this range, changes in activity of the hot start nucleic acid polymerase due to heat activation can be discriminatively measured.

When the product containing the hot start nucleic acid polymerase comprises a surfactant or a buffer component having absorbance such that the concentration of the hot start nucleic acid polymerase cannot be measured, the method further comprises, between steps (b) and (c), (b-1) diluting each of the heat-activated products by at least a 1/4-fold dilution.

The dilution of step (b-1) may be performed using a reaction buffer.

Step (c): <u>measuring an activity of each hot start nucleic acid polymerase contained in each of the heat-activated products for different times (**130**)</u>

Next, the method of the present invention comprises step (c) of measuring an activity of each hot start nucleic acid polymerase contained in each of the heat-activated products for different times. The activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization conditions to form an extended duplex, and a detectable signal is provided in a manner dependent on the extended duplex.

As used herein, the term "activity" referring to a hot start nucleic acid polymerase, refers to polymerization activity of the hot start nucleic acid polymerase.

The activity of each hot start nucleic acid polymerase contained in each of the products heat-activated for different times is measured from the intensity of a signal obtained by adding each of the products heat-activated for different times to the composition for activity assay of a nucleic acid polymerase and performing a nucleic acid polymerization reaction.

According to one embodiment of the present invention, in step (c), addition of each of the heat-activated products for different times to the composition for activity assay of a nucleic acid polymerase is performed by adding each of the heat-activated products for different times at different dilution factors. For example, each product may be diluted 100 fold, 200 fold, 300 fold, 400 fold, and 500 fold and then added to the composition for activity assay of a nucleic acid polymerase.

The composition for activity assay of a nucleic acid polymerase comprises a primer, and the primer is extended by the hot start nucleic acid polymerase contained in each of the heat-activated products under polymerization conditions to form an extended duplex, whereby a detectable signal is provided in a manner dependent on the extended duplex.

As used herein, the term "primer" refers to an oligonucleotide that can serve as an initiation point for synthesis of a primer extension product under conditions that induce synthesis of a primer extension product complementary to a nucleic acid strand (template), *i.e.*, in the presence of nucleotides and a polymerizing agent such as a DNA polymerase and under suitable temperature and pH conditions. In particular, a primer is a single-stranded deoxyribonucleotide molecule. The primer used in the present invention may comprise naturally occurring dNMPs (*i.e.*, dAMP, dGMP, dCMP, and dTMP), modified nucleotides, or non-natural nucleotides. In addition, a conventional primer may comprise ribonucleotides.

The primer should be sufficiently long to prime synthesis of an extension product in the presence of a polymerizing agent. A suitable length of the primer may be determined depending on a plurality of factors including temperature, application, and source of the primer. As used herein, the terms "annealing" or "priming" refer to apposition of an oligodeoxynucleotide or nucleic acid to a template nucleic acid, whereby the polymerase polymerizes nucleotides to form a nucleic acid molecule complementary to the template nucleic acid or a portion thereof.

The primer may have a length of 7-150 nucleotides, 10-120 nucleotides, 12-100 nucleotides, 14-80 nucleotides, 16-60 nucleotides, 18-40 nucleotides, 20-35 nucleotides, or 20-30 nucleotides.

According to one embodiment, the composition for activity assay of a nucleic acid polymerase further comprises (i) a template, (ii) a label, (iii) dNTPs, and (iv) a buffer.

(i) Template

In the polymerization reaction of the present invention, a nucleic acid polymerase polymerizes nucleotides by hybridizing to a template nucleic acid to form an extended duplex by generating a nucleic acid molecule complementary to the template nucleic acid.

Specifically, the template may be single-stranded M13mp18 DNA, or a synthesized template DNA to which a primer is linked, but is not limited thereto.

According to one embodiment of the present invention, the primer contained in the composition for activity assay of a nucleic acid polymerase comprises a dimer-forming primer pair that serves both as a primer and a template, and includes a first primer and a second primer, each of the first primer and the second primer comprises a 3'-dimer-forming portion, and a nucleotide sequence of the 3'-dimer-forming portion in the first primer is complementary to a nucleotide sequence of the 3'-dimer-forming portion in the second primer.

According to this embodiment, in the polymerization reaction, the dimer-forming primer pair is partially hybridized, and each primer serves as a template for the other primer. Nucleotides are polymerized by a nucleic acid polymerase to form nucleic acid molecules complementary to each primer, thereby forming an extended duplex.

As used herein, the term "extended duplex" refers to a duplex formed by an extension reaction in which a primer hybridized to a template (specifically, a primer serving as a template) is extended by a template-dependent nucleic acid polymerase.

As used herein, the term "dimer-forming primer" refers to a primer that can be partially hybridized to another dimer-forming primer (which is not, or serves as, a template) under specific conditions and extended in the presence of a nucleic acid polymerase.

As used herein, the term "dimer-forming primer pair" refers to a pair of primers, for example, a first primer and a second primer, that are partially hybridized to each other and can each be extended by polymerization activity of a nucleic acid polymerase.

As used herein, the terms "annealing" or "priming" with respect to a dimer-forming primer refer to apposition of a 3'-dimer-forming portion of the second primer to a 3'-dimer-forming portion of the first primer, whereby a nucleic acid polymerase polymerizes nucleotides to form a nucleic acid molecule complementary to a 5'-templating portion or some part thereof, thereby forming an extended duplex.

As used herein, the term "3'-dimer-forming portion" refers to a portion located at the 3' end of a dimer-forming primer that can hybridize with a 3'-dimer-forming portion of the other dimer-forming primer under specific hybridization conditions to form a dimer. That is, the 3'-dimer-forming portion refers to a portion at the 3' end of a dimer-forming primer having a nucleotide sequence capable of hybridizing with a 3'-dimer-forming portion of the other dimer-forming primer.

As used herein, the term "hybridization" refers to formation of a double-stranded nucleic acid by complementary single-stranded nucleic acids. Hybridization may occur when complementarity between two nucleic acid strands is perfect match, or may occur even when some mismatched bases are present. The degree of complementarity required for hybridization may vary depending on hybridization conditions, and may be controlled particularly by temperature.

Hybridization between a primer and a template, or hybridization between dimer-forming primers, may be performed under suitable hybridization conditions generally determined by optimization procedures. Conditions such as temperature, component concentration, number of hybridization and washing steps, buffer components and their pH and ionic strength may vary depending on various factors including primer length and GC content. For example, when using a relatively short primer or a dimer-forming primer pair having a short dimer-forming portion, it is preferable to select low stringency conditions. Detailed hybridization conditions may be found in Joseph Sambrook et al., Molecular Cloning: A Laboratory Manual, Cold Spring Harbor Laboratory Press, Cold Spring Harbor, N.Y. (2001), and M. L. M. Anderson, Nucleic Acid Hybridization, Springer-Verlag New York Inc., N.Y. (1999).

The terms "annealing" and "hybridization" are used interchangeably herein.

The primer has a hybridizable nucleotide sequence complementary to a template.

The first primer has a 3'-dimer-forming portion at its 3' end, the second primer has a 3'-dimer-forming portion at its 3' end, and the nucleotide sequence of the 3'-dimer-forming portion of the first primer is complementary to the nucleotide sequence of the 3'-dimer-forming portion of the second primer.

As used herein, the term "complementary" refers to being sufficiently complementary such that a primer hybridizes to a template or a 3'-dimer-forming portion of the first primer selectively hybridizes to a 3'-dimer-forming portion of the second primer under predetermined annealing or stringency conditions, and includes both substantially complementary and perfectly complementary, and more specifically refers to perfectly complementary.

The length of each 3'-dimer-forming portion of the first primer and the second primer may be 3-50 nucleotides, 5-45 nucleotides, 8-40 nucleotides, 10-35 nucleotides, 12-35 nucleotides, 14-30 nucleotides, or 15-25 nucleotides.

Each of the first primer and the second primer comprises not only the 3'-dimer-forming portion but also a 5'-templating portion.

As used herein, the term "5'-templating portion" of a dimer-forming primer refers to a remaining portion at the 5' end of the primer excluding the 3'-dimer-forming portion. The 5'-templating portion functions as a template for extension of the other dimer-forming primer hybridized thereto. The 5'-templating portion of a dimer-forming primer has a nucleotide sequence non-complementary to the 3'-dimer-forming portion of the other dimer-forming primer.

As used herein, the term "non-complementary" means sufficiently non-complementary such that a primer does not selectively hybridize to a template under predetermined annealing conditions or stringency conditions, and includes both "substantially non-complementary" and "perfectly non-complementary," and in particular means perfectly non-complementary. For example, with respect to a 5'-templating portion of a dimer-forming primer, the term "non-complementary" means that the 5'-templating portion of the dimer-forming primer is sufficiently non-complementary such that it does not selectively hybridize to the entire sequence of the other dimer-forming primer under predetermined annealing conditions or stringency conditions, and includes both "substantially non-complementary" and "perfectly non-complementary," and in particular means perfectly non-complementary.

The length of each 5'-templating portion of the first primer and the second primer may be 4-100 nucleotides, 6-90 nucleotides, 8-80 nucleotides, 10-70 nucleotides, 12-60 nucleotides, 14-50 nucleotides, 15-40 nucleotides, 15-30 nucleotides, or 20-30 nucleotides.

The dimer-forming primer or the dimer-forming primer pair is as disclosed in WO 2019/045532, and details regarding the dimer-forming primer or the dimer-forming primer pair are incorporated herein by reference to the foregoing document.

(ii) Label

The composition for activity assay of a nucleic acid polymerase may comprise a label to provide a detectable signal in a manner dependent on formation of an extended duplex.

As used herein, the phrase "a detectable signal is provided in a manner dependent on the extended duplex" means that a detectable signal is provided directly or indirectly from the presence of the extended duplex, and specifically, the detectable signal may be provided from the extended duplex itself.

More specifically, in the present invention, an intercalating label capable of providing a signal indicating the extent of formation of an extended duplex by a nucleic acid polymerase may be used. The intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™13, SYTO™15, SYTO™16, SYTO™20, SYTO™23, TOTO™-3, YOYO™3, GelStar™, thiazole orange, PicoGreen®, EvaGreen®, and LAM Fluorescent Dye.

Intercalating labels specifically intercalate into double-stranded nucleic acid molecules to generate a signal. As formation of an extended duplex increases, fluorescence intensity from a single fluorescent label increases. Accordingly, changes in the signal that increase with formation of an extended duplex can be detected in real time.

(iii) dNTP

The composition for activity assay of a nucleic acid polymerase may comprise dNTPs, which are building blocks of DNA or RNA to be synthesized. The dNTPs may comprise a mixture of four deoxyribonucleotides, *i.e.*, dATP, dCTP, dGTP, and dTTP (or dUTP).

(iv) Buffer

The composition for activity assay of a nucleic acid polymerase may comprise a buffer to provide a pH environment for optimal activity of the nucleic acid polymerase and to prevent abrupt pH changes due to temperature variations and chemical effects during the polymerization reaction. Examples of the buffer include Tris-HCl.

According to another embodiment of the present invention, the composition for activity assay of a nucleic acid polymerase further comprises (v) a salt and (vi) an additive.

(v) Salt

The composition for activity assay of a nucleic acid polymerase may comprise a salt to help stabilize activity of the nucleic acid polymerase. The salt may be a magnesium or potassium salt, for example, $MgCl_2$, $MgSO_4$, or KCl. The salt may be added at various concentrations known in the art.

(vi) Additive

The composition for activity assay of a nucleic acid polymerase may comprise various additives to stabilize the nucleic acid polymerase. Examples of the additive include BSA, DMSO, betaine, KCl, and a non-ionic surfactant (*e.g.*, Tween 20 and Triton X-100).

The nucleic acid polymerization reaction may include an isothermal polymerization reaction, PCR, quantitative real-time PCR (qPCR), melting curve analysis, high resolution melting analysis, sequencing, quantitative fluorescent PCR, multiplex PCR, digital PCR (dPCR), whole genome amplification (WGA), or reverse transcription PCR (RT-PCR).

According to one embodiment of the present invention, the nucleic acid polymerization reaction is performed at 35°C to 75°C for 10 to 60 minutes.

As used herein, the term "polymerization conditions" refers to the presence of components required for polymerization and suitable temperature and pH conditions. The term "polymerization conditions" also refers to conditions for separate steps included in the polymerization reaction, such as primer annealing/hybridization and primer extension. Such conditions may vary depending on various factors including primer length and GC content, and the length and GC content of a hybridization portion. Detailed conditions may be found in the literature [Joseph Sambrook et al., Molecular Cloning: A Laboratory Manual, Cold Spring Harbor Laboratory Press, Cold Spring Harbor, N.Y. (2001); and M. L. M. Anderson, Nucleic Acid Hybridization, Springer-Verlag New York Inc., N.Y. (1999)].

According to one embodiment of the present invention, the activity of the hot start nucleic acid polymerase measured in step (c) is provided from a slope measured in a range in which the intensity of a signal increases as a linear function depending on the concentration of the hot start nucleic acid polymerase contained in the product.

Specifically, the activity of the hot start nucleic acid polymerase contained in the product may be provided by measuring, for each concentration of the hot start nucleic acid polymerase contained in the product, a slope of a linear function portion of signal intensity over time (a differential value of time-signal intensity), and calculating a slope of a portion in which the differential value of time-signal intensity according to the concentration of the hot start nucleic acid polymerase contained in the product becomes a linear function.

According to one embodiment, the activity of the hot start nucleic acid polymerase measured in step (c) is activity per unit mass of the hot start nucleic acid polymerase or the activity per unit volume of the hot start nucleic acid polymerase.

According to one embodiment of the present invention, the hot start nucleic acid polymerase is hot start *Taq* DNA polymerase or hot start KlenTaq DNA polymerase, and the activity of the hot start nucleic acid polymerase in step (c) is provided as an amount of a nucleic acid polymerase determined by substituting the intensity of a signal into the following general formula 1:

**General formula 1** 1 unit (enzyme activity unit) = the amount of a nucleic acid polymerase required to represent an intensity of a signal showing polymerization by consuming 4.4 ± 0.56        General formula 1 nmole of dNTP for 30 minutes at 72°C.

According to one embodiment, the hot start nucleic acid polymerase is hot start *Taq* DNA polymerase or hot start KlenTaq DNA polymerase, and the activity of the hot start nucleic acid polymerase in step (c) is provided as a unit number of a nucleic acid polymerase determined by substituting the intensity of a signal into the following general formula 1:

**General formula 1** 1 unit (enzyme activity unit) = the amount of a nucleic acid polymerase required to represent an intensity of a signal showing polymerization by consuming 4.4 ± 0.56        General formula 1 nmole of dNTP for 30 minutes at 72°C.

Step (d): obtaining the activity of the hot start nucleic acid polymerase for different heat activation times as an activity profile of the hot start nucleic acid polymerase (**140**)

Finally, the method of the present invention comprises step (d) of obtaining the activity of the hot start nucleic acid polymerase for the different heat activation times as an activity profile of the hot start nucleic acid polymerase. The activity profile of the hot start nucleic acid polymerase indicates the performance of the hot start nucleic acid polymerase.

As used herein, the term "activity profile of a hot start nucleic acid polymerase" refers to a graphical representation illustrating a degree to which activity of a hot start nucleic acid polymerase, whose activity is initially suppressed, is restored by heat activation over time with respect to the heat activation time.

A major feature of the present invention is that performance of a hot start nucleic acid polymerase can be evaluated based on activity values or patterns (shapes) of the activity profile, and through such evaluation, it is possible to determine whether quality states among hot start nucleic acid polymerases are identical or not.

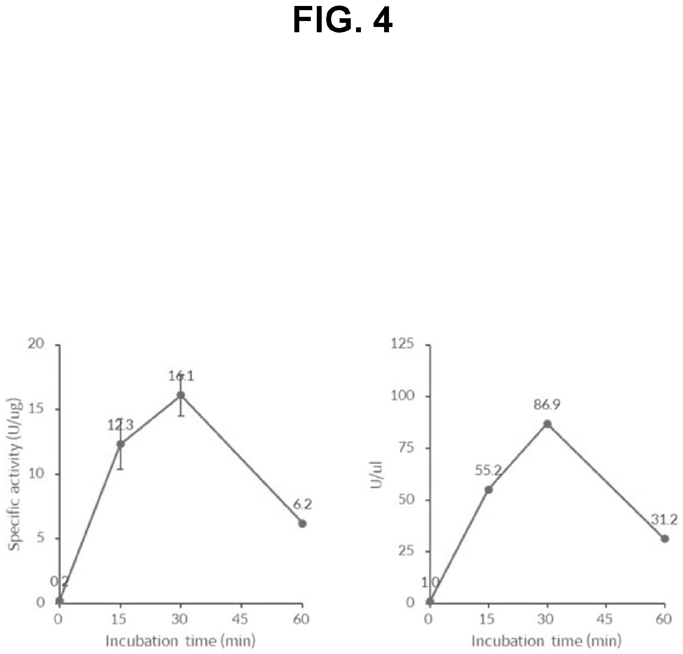

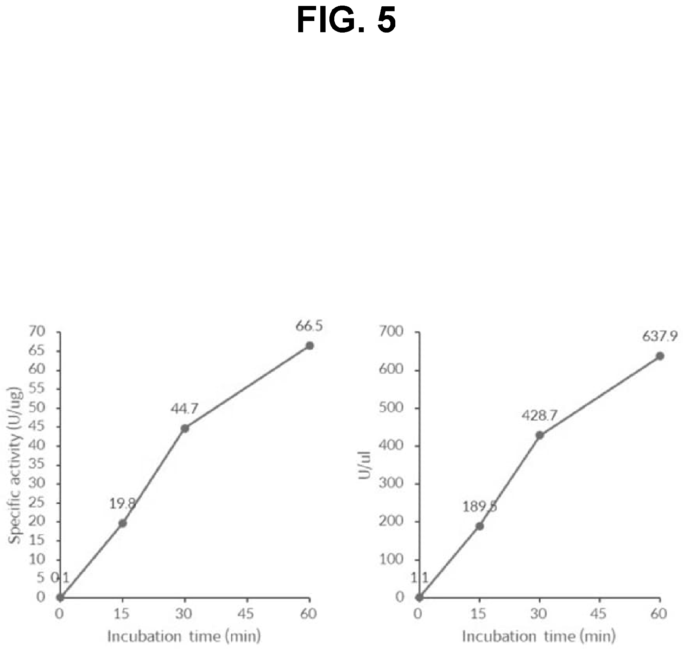

For example, FIG. 4 illustrates an activity profile of a hot start *Taq* DNA polymerase, and FIG. 5 illustrates an activity profile of a hot start KlenTaq polymerase, which is a mutant excluding an exonuclease activity domain of *Taq* DNA polymerase. As shown in FIGS. 4 and 5, changes (mutations) in properties of nucleic acid polymerases result in different activity values and different profile patterns through an activity profile even after hot start activation, indicating that such differences may affect PCR results differently.

According to one embodiment of the present invention, the method further comprises, after step (d), the following steps:

(d-1) obtaining a product containing a hot start nucleic acid polymerase obtained by a different obtaining process from the obtaining process of the product containing the hot start nucleic acid polymerase of step (a);

(d-2) performing steps (b) to (d) using the product obtained in step (d-1) to obtain an activity of the hot start nucleic acid polymerase contained in the product of step (d-1) for different heat activation times as an activity profile of the hot start nucleic acid polymerase contained in the product of step (d-1), wherein the activity profile of the hot start nucleic acid polymerase contained in the product of step (d-1) indicates performance of the hot start nucleic acid polymerase contained in the product of step (d-1); and

(d-3) comparing the activity profile of the hot start nucleic acid polymerase contained in the product of step (a) with the activity profile of the hot start nucleic acid polymerase contained in the product obtained in the obtaining process of step (d-1).

According to this embodiment, quality consistency among hot start nucleic acid polymerases for each production batch can be verified by comparing activity profiles of a hot start nucleic acid polymerase obtained from a first production process with those of a hot start nucleic acid polymerase obtained from a second production process.

**II. Method for Obtaining a QC-able Hot Start Nucleic Acid Polymerase (Second Aspect)**

In another aspect of the present invention, there is provided a method for obtaining a QC-able hot start nucleic acid polymerase, comprising:

(a) obtaining a product containing a hot start nucleic acid polymerase;
(b) aliquoting the product containing the hot start nucleic acid polymerase and performing heat activation on each of the aliquoted products for different times;
(c) measuring an activity of each hot start nucleic acid polymerase contained in each of the heat-activated products for different times; wherein the activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization conditions to form an extended duplex, and a detectable signal is provided in a manner dependent on the extended duplex;
(d) obtaining the activity of the hot start nucleic acid polymerase for the different heat activation times as an activity profile of the hot start nucleic acid polymerase, wherein the activity profile of the hot start nucleic acid polymerase indicates performance of the hot start nucleic acid polymerase; and
(e) providing the hot start nucleic acid polymerase for which the activity profile has been obtained.

The present invention relates to a method for obtaining a QC-able hot start nucleic acid polymerase by using the above-described method for evaluating performance of a hot start nucleic acid polymerase. Accordingly, the common descriptions among them are omitted in order to avoid undue redundancy leading to the complexity of this specification.

As used herein, the phrase "method for obtaining a QC-able nucleic acid polymerase" refers to a method of producing a nucleic acid polymerase that enables quality control (QC) by obtaining, evaluating, and monitoring an activity profile of a hot start nucleic acid polymerase, in which activity of the hot start nucleic acid polymerase initially suppressed is restored over time by heat activation performed at different times.

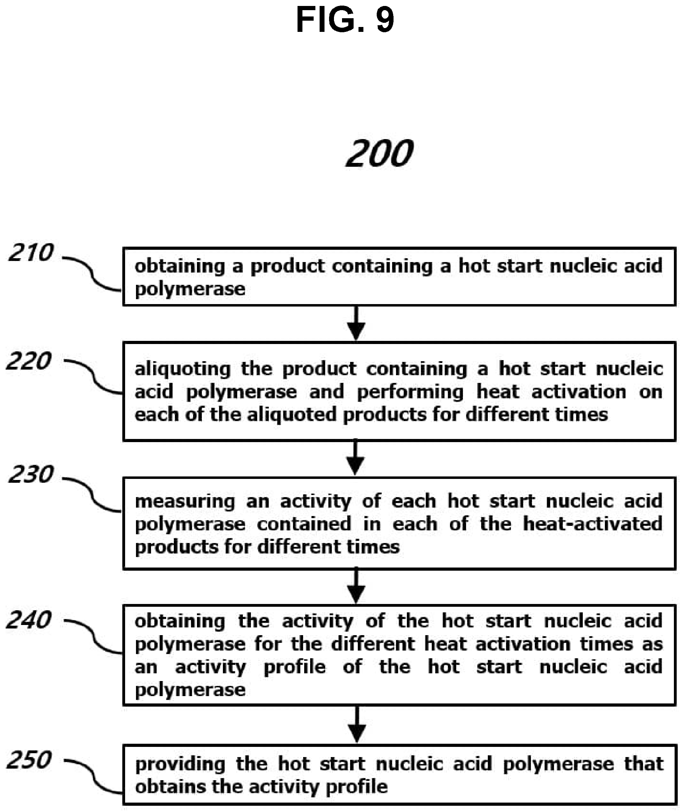

FIG. 9 is a flowchart illustrating processes of performing the method according to one embodiment of the present invention. The method of the present invention will be described in detail with reference to FIG. 9 as follows. Step (a): obtaining a product containing a hot start nucleic acid polymerase (**210**)

First, the method of the present invention comprises step (a) of obtaining a product containing a hot start nucleic acid polymerase.

According to one embodiment of the present invention, the hot start nucleic acid polymerase is a chemically modified hot start nucleic acid polymerase, an antibody-based hot start nucleic acid polymerase, or an aptamer-based hot start nucleic acid polymerase. Most specifically, the hot start nucleic acid polymerase is a chemically modified hot start nucleic acid polymerase.

According to one embodiment, the nucleic acid polymerase is a DNA polymerase.

According to a more specific embodiment of the present invention, the DNA polymerase is *Taq* DNA polymerase, KlenTaq DNA polymerase, Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase, or HIV reverse transcriptase.

According to one embodiment, the nucleic acid polymerase is prepared by a method comprising the following steps: (a-1) culturing host cells containing an expression construct for a nucleic acid polymerase; and (a-2) purifying the nucleic acid polymerase by applying a resultant of step (a-1) to column chromatography.

According to one embodiment of the present invention, the hot start nucleic acid polymerase of step (a) is a nucleic acid polymerase with inhibited polymerization activity. More specifically, the hot start nucleic acid polymerase is a nucleic acid polymerase whose polymerization activity is suppressed to 1 U/μg or less.

According to one embodiment, the method further comprises, after step (a), (a-3) diluting the product(s) to a concentration of 0.02 mg/mL to 2 mg/mL.

Step (b): <u>performing heat activation on each of the aliquoted products for different times (**220**)</u>

Next, the method of the present invention comprises step (b) of aliquoting the product containing the hot start nucleic acid polymerase and performing heat activation on each of the aliquoted products for different times.

According to one embodiment of the present invention, the heat activation of step (b) is performed on each of the aliquoted products for at least two time intervals selected from 0 to 60 minutes.

According to one embodiment, the heat activation of step (b) is performed at 90°C to 100°C.

According to one embodiment of the present invention, the heat activation of step (b) is performed on each of the aliquoted products at 90°C to 100°C for 0 minutes, 15 minutes, 30 minutes, and 60 minutes.

According to one embodiment, the method further comprises, between steps (b) and (c), (b-1) diluting each of the heat-activated products to a concentration of 6.25 ng/$\mu$L to 50 ng/$\mu$L.

Step (c): <u>measuring an activity of each hot start nucleic acid polymerase contained in each of the heat-activated products for different times (**230**)</u>

Next, the method of the present invention comprises step (c) of measuring an activity of each hot start nucleic acid polymerase contained in each of the heat-activated products for different times. The activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization conditions to form an extended duplex, and a detectable signal is provided in a manner dependent on the extended duplex.

According to one embodiment of the present invention, the activity of the hot start nucleic acid polymerase measured in step (c) is provided from a slope measured in a range in which the intensity of a signal increases as a linear function depending on a concentration of the hot start nucleic acid polymerase contained in the product.

According to one embodiment, the activity of the hot start nucleic acid polymerase measured in step (c) is activity per unit mass of the hot start nucleic acid polymerase or activity per unit volume of the hot start nucleic acid polymerase.

According to one embodiment of the present invention, the composition for activity assay of a nucleic acid polymerase further comprises (i) a template, (ii) a label, (iii) dNTPs, and (iv) a buffer.

According to one embodiment, the primer contained in the composition for activity assay of a nucleic acid polymerase comprises a first primer and a second primer, which are a pair of dimer-forming primers that serve both as a primer and a template, each of the first primer and the second primer comprises a 3'-dimer-forming portion, and a nucleotide sequence of the 3'-dimer-forming portion in the first primer is complementary to a nucleotide sequence of the 3'-dimer-forming portion in the second primer.

According to one embodiment of the present invention, in step (c), the addition of each of the heat-activated products for different times to the composition for activity assay of a nucleic acid polymerase is performed by adding each of the heat-activated products for different times at different dilution factors to the composition for activity assay of a nucleic acid polymerase.

According to one embodiment, the nucleic acid polymerization reaction is performed at 35°C to 75°C for 10 to 60 minutes.

According to a more specific embodiment of the present invention, the label is an intercalating label. More specifically, the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™13, SYTO™15, SYTO™16, SYTO™20, SYTO™23, TOTO™-3, YOYO™3, GelStar™, thiazole orange, PicoGreen®, EvaGreen®, and LAM Fluorescent Dye.

According to one embodiment, the hot start nucleic acid polymerase is hot start *Taq* DNA polymerase or hot start KlenTaq DNA polymerase, and the activity of the hot start nucleic acid polymerase in step (c) is provided as an amount of a nucleic acid polymerase determined by substituting the intensity of a signal into the following general formula 1:

**General formula 1** 1 unit (enzyme activity unit) = the amount of a nucleic acid polymerase required to represent an intensity of a signal showing polymerization by consuming 4.4 $\pm$ 0.56  General formula 1 nmole of dNTP for 30 minutes at 72°C.

According to one embodiment of the present invention, the hot start nucleic acid polymerase is hot start *Tag* DNA polymerase or hot start KlenTaq DNA polymerase, and the activity of the hot start nucleic acid polymerase in step (c) is provided as a unit number of a nucleic acid polymerase determined by substituting the intensity of a signal into the following general formula 1:

**General formula 1** 1 unit (enzyme activity unit) = the amount of a nucleic acid polymerase required to represent an intensity of a signal showing polymerization by consuming 4.4 ± 0.56    General formula 1 nmole of dNTP for 30 minutes at 72°C.

Step (d): <u>obtaining an activity of the hot start nucleic acid polymerase for different heat activation times as an activity profile of the hot start nucleic acid polymerase (**240**)</u>

Next, the method of the present invention comprises step (d) of obtaining the activity of the hot start nucleic acid polymerase for the different heat activation times as an activity profile of the hot start nucleic acid polymerase. The activity profile of the hot start nucleic acid polymerase indicates the performance of the hot start nucleic acid polymerase.

According to one embodiment of the present invention, the method further comprises, after step (d), the following steps:

(d-1) obtaining a product containing a hot start nucleic acid polymerase obtained by a different obtaining process from the obtaining process of the product containing the hot start nucleic acid polymerase of step (a);
(d-2) performing steps (b) to (d) using the product obtained in step (d-1) to obtain an activity of the hot start nucleic acid polymerase contained in the product of step (d-1) for different heat activation times as an activity profile of the hot start nucleic acid polymerase contained in the product of step (d-1), wherein the activity profile of the hot start nucleic acid polymerase contained in the product of step (d-1) indicates performance of the hot start nucleic acid polymerase contained in the product of step (d-1); and
(d-3) comparing the activity profile of the hot start nucleic acid polymerase contained in the product of step (a) with the activity profile of the hot start nucleic acid polymerase contained in the product obtained in the obtaining process of step (d-1).

Step (e): <u>providing the hot start nucleic acid polymerase that obtains the activity profile (**250**)</u>

Finally, the method of the present invention comprises step (e) of providing the hot start nucleic acid polymerase that obtains the activity profile.

In this step, the hot start nucleic acid polymerase for which the activity profile has been obtained is provided.

**III. Method for Providing Blended Hot Start Nucleic Acid Polymerases that Mimic a Performance of a Target Hot Start Nucleic Acid Polymerase (Third Aspect)**

In still another aspect of the present invention, there is provided a method for providing blended hot start nucleic acid polymerases that mimic a performance of a target hot start nucleic acid polymerase, comprising:

(a) obtaining i) a product containing a target hot start nucleic acid polymerase, and ii) products each containing at least two hot start nucleic acid polymerases to be blended;
(b) i) aliquoting the product containing the target hot start nucleic acid polymerase and performing heat activation on each of the aliquoted products for different times, and ii) aliquoting each of the products each containing the at least two hot start nucleic acid polymerases to be blended and performing heat activation on each of the aliquoted products for different times, wherein the heat activation time of i) is the same as the heat activation time of ii);
(c) measuring an activity of each target hot start nucleic acid polymerase contained in each of the heat-activated products for different times in i) of step (b), and measuring an activity of each of the at least two hot start nucleic acid polymerases to be blended contained in each of the heat-activated products for different times in ii) of step (b); wherein the activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization conditions to form an extended duplex, and a detectable signal is provided in a manner dependent on the extended duplex;
(d) obtaining the activity of the target hot start nucleic acid polymerase for the different heat activation times in i) of step (b) as an activity profile of the target hot start nucleic acid polymerase, and obtaining the activity of the at least two hot start nucleic acid polymerases to be blended for the different heat activation times in ii) of step (b) as activity profiles of the at least two hot start nucleic acid polymerases to be blended; wherein the activity profile of the target hot start nucleic acid polymerase indicates performance of the target hot start nucleic acid polymerase, and each of the activity profiles of the at least two hot start nucleic acid polymerases to be blended indicates performance of each of the at least two hot start nucleic acid polymerases to be blended;

(e) providing an amount of each of the at least two hot start nucleic acid polymerases to be blended by using the performance of the target hot start nucleic acid polymerase and the performance of the at least two hot start nucleic acid polymerases to be blended to mimic the performance of the target hot start nucleic acid polymerase; and

(f) providing the at least two hot start nucleic acid polymerases blended in the amounts provided in step (e) as the blended hot start nucleic acid polymerases that mimic the performance of the target hot start nucleic acid polymerase.

The present invention relates to a method for providing blended hot start nucleic acid polymerases that mimic a performance of a target hot start nucleic acid polymerase by using the above-described method for evaluating performance of a hot start nucleic acid polymerase. Accordingly, the common descriptions among them are omitted in order to avoid undue redundancy leading to the complexity of this specification.

As used herein, the term "target hot start nucleic acid polymerase" refers to a target hot start nucleic acid polymerase whose performance is intended to be mimicked or replicated by blending (mixing or combining) at least two hot start nucleic acid polymerases.

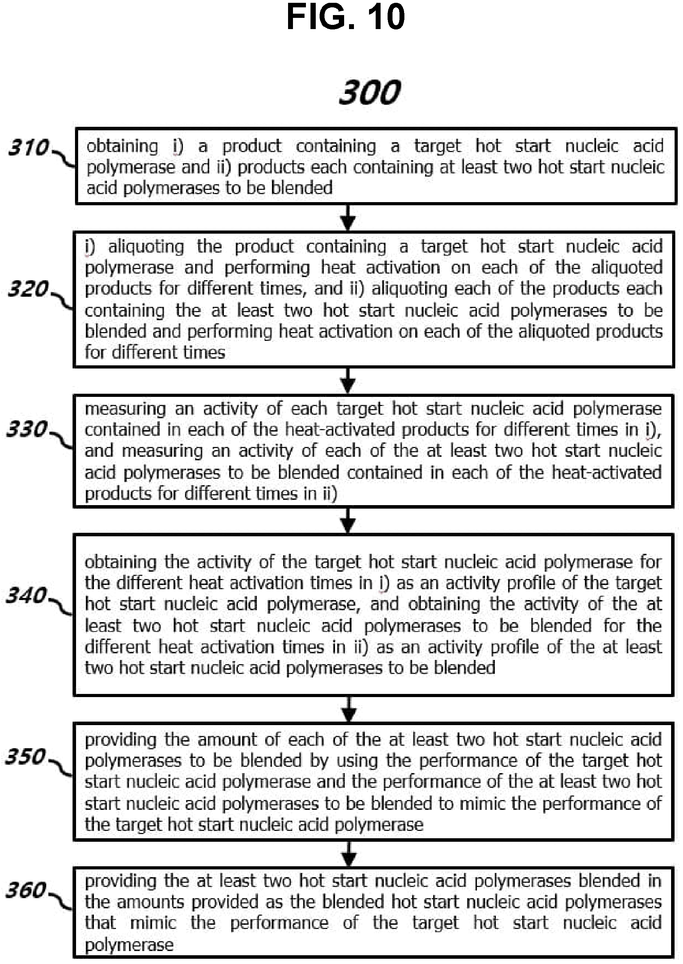

FIG. 10 is a flowchart illustrating processes of performing the method of this invention according to one embodiment of the present invention. The method of the present invention will be described with reference to FIG. 10 as follows. Step (a): obtaining products containing hot start nucleic acid polymerases (**310**)

First, the method of the present invention comprises step (a) of obtaining i) a product containing a target hot start nucleic acid polymerase, and ii) products each containing at least two hot start nucleic acid polymerases to be blended.

According to one embodiment of the present invention, the number of the hot start nucleic acid polymerases to be blended is up to 10. More specifically, the hot start nucleic acid polymerases to be blended are selected from 2 to 8, more specifically from 2 to 5, and even more specifically 2 or 3.

According to one embodiment, the hot start nucleic acid polymerase is a chemically modified hot start nucleic acid polymerase, an antibody-based hot start nucleic acid polymerase, or an aptamer-based hot start nucleic acid polymerase. Most specifically, the hot start nucleic acid polymerase is a chemically modified hot start nucleic acid polymerase.

According to one embodiment of the present invention, the nucleic acid polymerase is a DNA polymerase.

According to a more specific embodiment of the present invention, the DNA polymerase is *Taq* DNA polymerase, KlenTaq DNA polymerase, a Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase, or HIV reverse transcriptase.

According to one embodiment, the nucleic acid polymerase is prepared by a method comprising the following steps: (a-1) culturing a host cell comprising an expression construct for a nucleic acid polymerase; and (a-2) purifying the nucleic acid polymerase by applying the resultant of step (a-1) to column chromatography.

According to one embodiment of the present invention, the hot start nucleic acid polymerase of step (a) is a nucleic acid polymerase whose polymerization activity is suppressed. More specifically, the hot start nucleic acid polymerase is a nucleic acid polymerase whose polymerization activity is suppressed to 1 U/$\mu$g or less.

According to one embodiment, the method further comprises, after step (a), (a-3) diluting the product(s) to a concentration of 0.02 mg/mL to 2 mg/mL.

Step (b): performing heat activation on each of the aliquoted products for different times (**320**)

Next, the method of the present invention comprises step (b) of i) aliquoting the product containing the target hot start nucleic acid polymerase and performing heat activation on each of the aliquoted products for different times, and ii) aliquoting each of the products each containing at least two hot start nucleic acid polymerases to be blended and performing heat activation on each of the aliquoted products for different times, wherein the heat activation time of i) is the same as the heat activation time of ii).

According to one embodiment of the present invention, the heat activation of step (b) is performed on each of the aliquoted products for at least two time intervals selected from 0 to 60 minutes.

According to one embodiment, the heat activation of step (b) is performed at 90°C to 100°C.

According to one embodiment of the present invention, the heat activation of step (b) is performed on each of the aliquoted products at 90°C to 100°C for 0 minutes, 15 minutes, 30 minutes, and 60 minutes.

According to one embodiment, the method further comprises, between steps (b) and (c), (b-1) diluting each of the heat-activated products to a concentration of 6.25 ng/$\mu$L to 50 ng/$\mu$L.

Step (c): measuring an activity of each hot start nucleic acid polymerase contained in each of the heat-activated products for different times (**330**)

Next, the method of the present invention comprises step (c) of measuring an activity of each target hot start nucleic acid polymerase contained in each of the heat-activated products for different times in i) of step (b), and measuring an activity of each of the at least two hot start nucleic acid polymerases to be blended contained in each of the heat-activated products for different times in ii) of step (b). The activity is measured from an intensity of a signal measured by adding each of the heat-activated products for different times to a composition for activity assay of a nucleic acid polymerase and performing a nucleic acid polymerization reaction, the composition for activity assay of a nucleic acid polymerase comprises a primer, the primer is extended by the hot start nucleic acid polymerase contained in each of the heat-activated products under polymerization conditions to form an extended duplex, and a detectable signal is provided in a manner dependent on the extended duplex.

According to one embodiment of the present invention, the activity of the hot start nucleic acid polymerase measured in step (c) is provided from a slope measured in a range in which the signal intensity increases as a linear function according to a concentration of the hot start nucleic acid polymerase contained in the product.

According to one embodiment, the activity of the hot start nucleic acid polymerase measured in step (c) is an activity per unit mass of the hot start nucleic acid polymerase or an activity per unit volume of the hot start nucleic acid polymerase.

According to one embodiment of the present invention, the composition for activity assay of a nucleic acid polymerase further comprises (i) a template, (ii) a label, (iii) dNTPs, and (iv) a buffer.

According to one embodiment, the primer contained in the composition for activity assay of a nucleic acid polymerase comprises a first primer and a second primer, which are a pair of dimer-forming primers that serve both as a primer and a template; wherein the first primer and the second primer each comprises a 3'-dimer-forming portion, and a nucleotide sequence of the 3'-dimer-forming portion in the first primer is complementary to a nucleotide sequence of the 3'-dimer-forming portion in the second primer.

According to one embodiment of the present invention, in step (c), the addition of each of the heat-activated products for different times to a composition for activity assay of a nucleic acid polymerase is performed by adding each of the heat-activated products for different times to a composition for activity assay of a nucleic acid polymerase by dilution factor.

According to one embodiment, the nucleic acid polymerization reaction is performed at 35°C to 75°C for 10 to 60 minutes.

According to a more specific embodiment of the present invention, the label is an intercalating label. More specifically, the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, YO-PRO™1, SYTO™11, SYTO™13, SYTO™15, SYTO™16, SYTO™20, SYTO™23, TOTO™-3, YOYO™3, GelStar™, thiazole orange, PicoGreen®, EvaGreen®, and LAM Fluorescent Dye.

According to one embodiment of the present invention, the hot start nucleic acid polymerase is a hot start *Taq* DNA polymerase or a hot start KlenTaq DNA polymerase, and the activity of the hot start nucleic acid polymerase in step (c) is provided as an amount of the nucleic acid polymerase determined by substituting the intensity of a signal into the following general formula 1:

**General formula 1** 1 unit (enzyme activity unit) = an amount of a nucleic acid polymerase required to exhibit a signal intensity showing polymerization by consuming 4.4 ± 0.56 nmoles of      General formula 1
dNTPs at 72°C for 30 minutes.

According to one embodiment, the hot start nucleic acid polymerase is a hot start *Taq* DNA polymerase or a hot start KlenTaq DNA polymerase, and the activity of the hot start nucleic acid polymerase in step (c) is provided as a unit number of the nucleic acid polymerase determined by substituting the signal intensity into the following general formula 1:

**General formula 1** 1 unit (enzyme activity unit) = an amount of a nucleic acid polymerase required to exhibit a signal intensity showing polymerization by consuming 4.4 ± 0.56 nmoles of      General formula 1
dNTPs at 72°C for 30 minutes.

Step (d): <u>obtaining the activity of the hot start nucleic acid polymerases for the different heat activation times as an activity profile of the hot start nucleic acid polymerase (**340**)</u>

Next, the method of the present invention comprises step (d) of obtaining the activity of the target hot start nucleic acid polymerase for the different heat activation times in i) of step (b) as an activity profile of the target hot start nucleic acid polymerase, and obtaining the activity of the at least two hot start nucleic acid polymerases to be blended for the different heat activation times in ii) of step (b) as an activity profile of the at least two hot start nucleic acid polymerases to be blended. The activity profile of the target hot start nucleic acid polymerase indicates a performance of the target hot start nucleic acid polymerase, and each of the activity profiles of the at least two hot start nucleic acid polymerases to be blended indicates a performance of each of the at least two hot start nucleic acid polymerases to be blended.

Step (e): providing the amount of each of the at least two hot start nucleic acid polymerases to be blended by using the performance of the target hot start nucleic acid polymerase and the performance of the at least two hot start nucleic acid polymerases to be blended to mimic the performance of the target hot start nucleic acid polymerase (350)

Next, the method of the present invention comprises step (e) of providing the amount of each of the at least two hot start nucleic acid polymerases to be blended by using the performance of the target hot start nucleic acid polymerase and the performance of the at least two hot start nucleic acid polymerases to be blended to mimic the performance of the target hot start nucleic acid polymerase.

A major feature of the present invention is that the amount of each of the at least two hot start nucleic acid polymerases to be blended, which mimic the performance of the target hot start nucleic acid polymerase, can be provided by using activity profiles indicating the performance of the target hot start nucleic acid polymerase and the at least two hot start nucleic acid polymerases to be blended.

According to a more specific embodiment of the present invention, the step (e) is a step of providing the amount of each of the at least two hot start nucleic acid polymerases to be blended by substituting the performance of the target hot start nucleic acid polymerase and the performance of at least two hot start nucleic acid polymerases to be blended to mimic the performance of the target hot start nucleic acid polymerase into a linear algebraic equation.

In this embodiment, activity values of the activity profiles indicating the performance of the hot start nucleic acid polymerases are substituted into the linear algebraic equation to provide the amount of each of the at least two hot start nucleic acid polymerases to be blended that mimics the performance of the target hot start nucleic acid polymerase.

In this specification, a linear algebraic equation refers to a simultaneous equation in which all variables are first-order term.

According to a more specific embodiment of the present invention, the linear algebraic equation is expressed by the following formula 1:

## Formula 1

$$[A]_{nxn}[X]_{nx1} = [B]_{nx1}$$

wherein the matrix [A] represents the performance of at least two hot start nucleic acid polymerases to be blended, n of the row element showing the size of the matrix in the matrix [A] represents the order of heat activation times and n of the column element in the matrix [A] represents hot start nucleic acid polymerases to be blended; the matrix [X] represents the amount of at least two hot start nucleic acid polymerases to be blended, and n of the row element showing the size of the matrix in the matrix [X] represents hot start nucleic acid polymerases to be blended; and the matrix [B] represents the performance of a target hot start nucleic acid polymerase, and n of the row element showing the size of the matrix in the matrix [B] represents the order of heat activation times.

According to a most specific embodiment of the present invention, the formula 1 is expressed by the following formula 2:

## Formula 2

$$\begin{bmatrix} a_{11} & a_{12} & \cdots & a_{1n} \\ a_{21} & a_{22} & \cdots & a_{2n} \\ \vdots & \vdots & \vdots & \vdots \\ a_{n1} & a_{n2} & \cdots & a_{nn} \end{bmatrix} \begin{bmatrix} x_1 \\ x_2 \\ \vdots \\ x_n \end{bmatrix} = \begin{bmatrix} b_1 \\ b_2 \\ \vdots \\ b_n \end{bmatrix}$$

wherein the matrix element $a$ represents the activity of at least two hot start nucleic acid polymerases to be blended, the row element $n$ in the matrix element $a$ represents the order of heat activation times, the column element $n$ in the matrix element $a$ represents hot start nucleic acid polymerases to be blended; the matrix element $x$ represents the amount of at least two hot start nucleic acid polymerases to be blended, the row element $n$ in the matrix element $x$ represents hot start nucleic acid polymerases to be blended; and the matrix element b represents the activity of a target hot start nucleic acid polymerase, and the row element $n$ in the matrix element $b$ represents the order of heat activation times.

In formula 1 and formula 2, n of the row components representing the hot start nucleic acid polymerases to be blended in the matrix [X] and the matrix element x is up to 10, more specifically selected from 2 to 8, more specifically from 2 to 5, and most specifically 2 or 3.

In formula 1 and formula 2, n of the row elements or components representing the order of heat activation times in the matrices [A] and [B], and in the matrix elements a and b, is a natural number. For example, 1) when heat activation is performed for 0 minutes and 60 minutes, the order of the heat activation time of 0 minutes is 1, and the order of the heat activation time of 60 minutes is 2; 2) when heat activation is performed for 0 minutes, 15 minutes, and 60 minutes, the order of the heat activation times of 0 minutes, 15 minutes, and 60 minutes are 1, 2, and 3, respectively; and 3) when heat activation is performed for 0 minutes, 15 minutes, 45 minutes, and 60 minutes, the order of the heat activation times of 0 minutes, 15 minutes, 30 minutes, and 60 minutes are 1, 2, 3, and 4, respectively.

According to one embodiment of the present invention, n of the row elements or components representing the order of heat activation times is selected from 1 to 10, more specifically from 2 to 8, more specifically from 2 to 5, and most specifically 2 or 3.

According to one embodiment, the performances of the at least two hot start nucleic acid polymerases to be blended and the target hot start nucleic acid polymerase are activities of the at least two hot start nucleic acid polymerases to be blended and the target hot start nucleic acid polymerase.

In formula 1 and formula 2, when the activities of the at least two hot start nucleic acid polymerases to be blended are activities per unit mass (Unit) and the activity of the target hot start nucleic acid polymerase is an activity (Unit), the amounts of the at least two hot start nucleic acid polymerases to be blended are masses; and when the activities of the at least two hot start nucleic acid polymerases to be blended are activities per unit mass (Unit) and the activity of the target hot start nucleic acid polymerase is an activity per reaction (Unit), the amounts of the at least two hot start nucleic acid polymerases to be blended are masses per reaction.

Step (f): providing the at least two hot start nucleic acid polymerases blended in the amounts provided as the blended hot start nucleic acid polymerases that mimic the performance of the target hot start nucleic acid polymerase (**360**)

Finally, the method of the present invention comprises step (f) of providing the at least two hot start nucleic acid polymerases blended in the amounts provided in step (e) as the blended hot start nucleic acid polymerases that mimic the performance of the target hot start nucleic acid polymerase.

By this method, the blended hot start nucleic acid polymerases that mimic the performance of the target hot start nucleic acid polymerase can be provided.

## EFFECTS OF THE INVENTION

The features and advantages of the present invention are summarized as follows:

(a) Conventionally, since a hot start nucleic acid polymerase is in a state in which its activity is suppressed, there has been a problem in that its activity is measured as extremely low or is not measured at all by general methods for measuring activity of a nucleic acid polymerase.

(b) The present invention enables evaluation of performance of a hot start nucleic acid polymerase and provision of a QC-able hot start nucleic acid polymerase by constructing an activity profile of the hot start nucleic acid polymerase with respect to heat activation time using a method for measuring activity (unit) of a nucleic acid polymerase that does not use radioactive isotopes.

(c) Further, the present invention enables provision of a blended nucleic acid polymerase that mimics performance of a target hot start nucleic acid polymerase by using the activity profile of the hot start nucleic acid polymerase with respect to heat activation time.

## BRIEF DESCRIPTION OF THE DRAWINGS

FIG. 1 is a flowchart illustrating processes of performing the method according to one embodiment of the present invention.

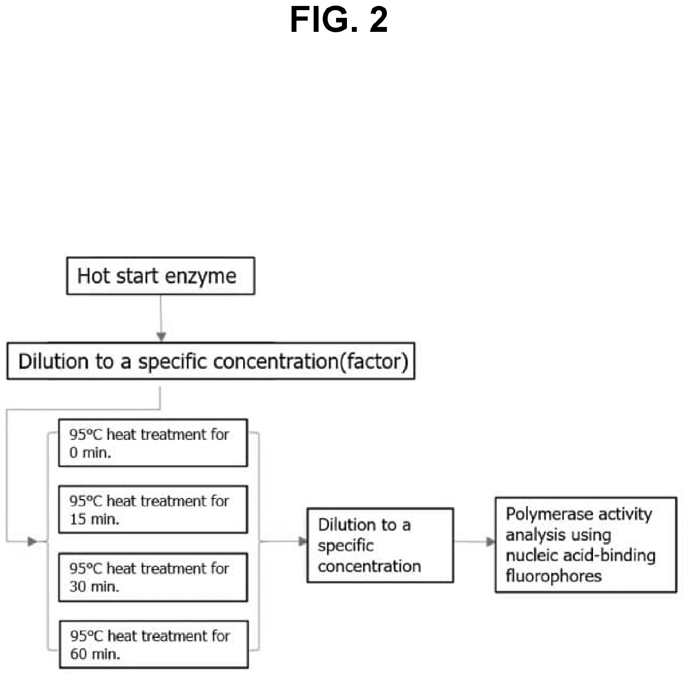

FIG. 2 illustrates an analysis flow chart of a hot start nucleic acid polymerase according to one embodiment of the present invention.

FIG. 3 shows a real-time fluorescence measurement graph for measuring polymerization activity according to one embodiment of the present invention.

FIG. 4 shows an activity profile obtained by analyzing activity of a hot start *Taq* DNA polymerase produced according to one embodiment of the present invention.

FIG. 5 shows an activity profile obtained by analyzing activity when a hot start is applied to a KlenTaq polymerase according to one embodiment of the present invention.

FIG. 6 is a graph showing activity of a hot start nucleic acid polymerase, for which concentration is difficult to determine, according to heat activation time, according to one embodiment of the present invention.

FIG. 7 shows an activity profile of the hot start nucleic acid polymerase of FIG. 6 according to one embodiment of the present invention.

FIG. 8 is a graph showing results of regression analysis between a Ct-based unit measurement method and a unit measurement method according to one embodiment of the present invention.

FIG. 9 is a flowchart illustrating processes of performing the method according to another embodiment of the present invention.

FIG. 10 is a flowchart illustrating processes of performing the method according to still another embodiment of the present invention.

FIG. 11 shows equations for calculating an amount (*e.g.*, mass, concentration, etc.) of hot start polymerases to be blended to mimic a target hot start polymerase by a linear algebraic equation (matrix) according to still another embodiment of the present invention.

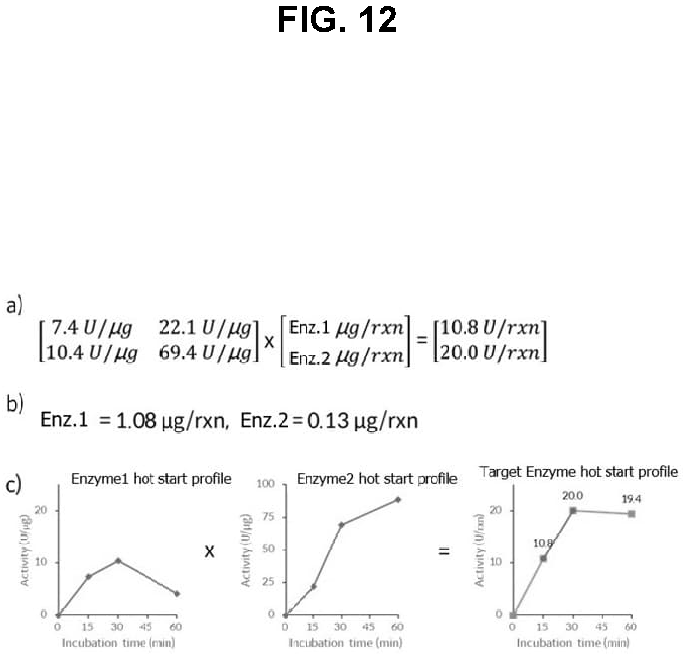

FIG. 12 (a) shows an equation obtained by substituting, into a linear algebraic equation (matrix), an activity value-per-reaction from an activity profile of a target hot start polymerase and activity values-per-unit mass from activity profiles of hot start polymerase 1 and hot start polymerase 2 to be blended, in order to determine amounts of arbitrary hot start polymerase 1 and hot start polymerase 2 to be blended that mimic performance of the target hot start polymerase. FIG. 12 (b) shows masses per reaction of hot start polymerase 1 and hot start polymerase 2 to be blended, calculated using the equation or formula of FIG. 12(a). FIG. 12 (c) shows activity profiles of hot start polymerase 1 and hot start polymerase 2 to be blended and an activity profile of the target hot start polymerase, wherein, at heat activation times of 15 minutes and 30 minutes in the activity profile of the target hot start polymerase, activity profiles of the blended hot start polymerases calculated by the equation are overlaid.

FIG. 13 is a graph showing that an activity profile of a blended hot start polymerase mixed in the amounts calculated in FIG. 12 is similar to an activity profile of the target hot start polymerase to be mimicked.

The present invention will now be described in further detail by examples. It would be obvious to those skilled in the art that these examples are intended to be more concretely illustrative and the scope of the present invention as set forth in the appended claims is not limited to or by the examples.

**Examples**

**Example 1: Measurement of Enzyme Activity Units Using a Fluorescence Amplification Method Based on Double-Stranded DNA Binding**

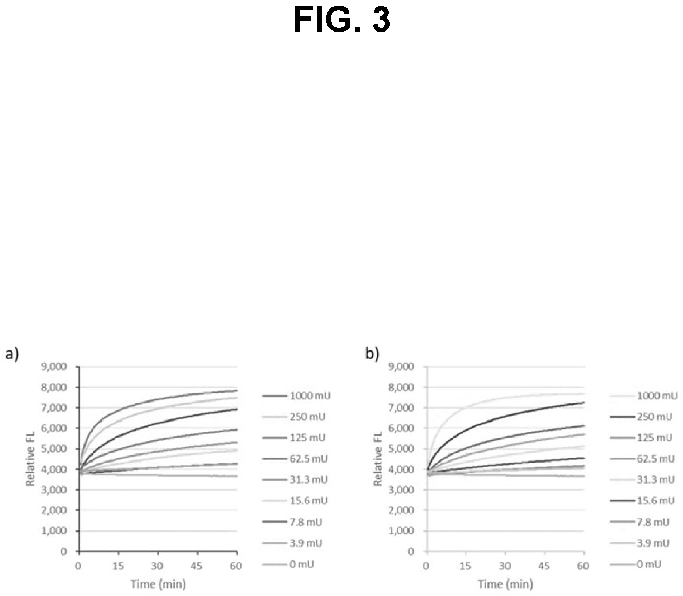

First, *Taq* DNA polymerase products from New England Biolabs (NEB) and Roche were selected as standard products. The concentration of each enzyme was first confirmed according to the literature (Jesse L. Montgomery et al., Analytical Biochemistry, 2013, 441(2), 133-139). In addition, in order to calculate an initial polymerization rate according to protein dilution factors, an EvaEZ™ product (containing EvaGreen® dye, a primed template, dNTPs, $MgCl_2$, and a Tris buffer) was used. Fluorescence changes were monitored at 30-second intervals for 60 minutes using a Bio-Rad CFX96-DR instrument (FIG. 3 (a)). Each experiment was repeated three times within the same set, and a total of seven sets were performed to accurately determine experimental error ranges.

The measured graph of FIG. 3 (x-axis - time, y-axis - fluorescence value) is drawn in the form of a curve that gradually becomes gentler as the dNTP, which is the substrate, is consumed and the dNTP gradually fails to participate in the 100% reaction for the enzyme. When the enzyme concentration is adjusted to specific dilution levels as described in Examples 1-1 or 1-2, the amount of dNTPs is sufficiently excessive relative to the enzyme during the initial within 10 minutes, such that the fluorescence intensity versus time exhibits a linear relationship. At this stage, the slope of the linear function (*i.e.*, the differential value of the time-fluorescence value graph) is measured. Since this value represents fluorescence increasing with DNA synthesis over time, it is directly correlated with the amount of dNTP consumption.

When this measured value is obtained at multiple concentrations where the initial reaction function is linear, as in Examples 1-1 or 1-2, the results can be expressed as a graph in which the x-axis represents enzyme concentration and the y-axis represents the differential value of time-fluorescence intensity. At this point, a linear function of the 'time-fluorescence derivative' with respect to the 'enzyme concentration' is again created. Here, the slope of this graph (derivative of enzyme concentration-the 'time-fluorescence derivative') ultimately becomes an indicator of the amount of dNTP consumption, which is directly proportional to the enzyme concentration. Therefore, this allows us to define a unit of polymerization activity of DNA polymerase as the amount of dNTP consumed per unit time at unit temperature.

As a result, it was confirmed that 1 Unit defined by NEB using a radioactive isotope-based method (consumption of 15 nmoles of dNTPs at 75°C for 30 minutes) corresponds to consumption of 4.4 $\pm$ 0.56 nmoles (95% confidence interval) of dNTPs at 72°C for 30 minutes using the EvaEZ™ product. This confirmation ensured the reliability of the unit measurement method despite a change in the measurement approach of standard products. It was also confirmed that the same level of confidence was achieved with Roche's product under the same conditions (consuming 10 nmole of dNTP for 30 minutes at 75°C).

Example 1-1: Preparation of Reagents for Selection of Standard Products

For selection of standard products, a commercially available *Taq* DNA polymerase at 5 U/$\mu$L was diluted using 10$\times$ ThermoPol reaction buffer (New England Biolabs) and ultrapure water to prepare concentrations of 1000, 500, 250, 125, 62.5, 31.3, 15.6, 7.8, and 3.9 mU/$\mu$L. Each preparation was mixed at a volume ratio of 10:1:9 (EvaEZ reagent : enzyme : ultrapure water). All procedures were performed on ice to maintain a low temperature (FIG. 3 a)). Similarly, Roche *Taq* DNA polymerase at 5 U/$\mu$L was diluted in the same manner to prepare reagents (FIG. 3 b)).

Example 1-2: Reaction of Reagents

To observe kinetic reactions, a Bio-Rad CFX96 system, which enables real-time fluorescence measurement with temperature control, was prepared. Reagents were aliquoted into 8-strip PCR tubes at 20 $\mu$L per tube, and reactions were carried out at 72 °C, with fluorescence measured at 30-second intervals. From the acquired data, the slope of the region exhibiting an initial linear function was calculated.

Example 1-3: Measurement of Specific Activity of 100% Pure *Taq* DNA Polymerase

Through the experiments of Example 1-2, when a reagent consistently produced the same value despite changes in instrument, operator, or reagent batches, the reagent was defined as a standard product. This standard product was subjected to buffer exchange and concentration using an Amicon Ultra centrifugal filter unit (30 kDa cut-off), followed by SDS-PAGE analysis. Bovine Serum Albumin (BSA) standards at concentrations ranging from 0.1 mg/mL to 1 mg/mL were loaded in equal volumes on the same SDS-PAGE gel. Based on these data, the actual amount of enzyme in the standard product was quantified, and the specific activity of the *Taq* DNA polymerase was calculated.

**Example 2: Preparation of Polymerase**

*Taq* DNA polymerase was expressed by culturing *Escherichia* coli transformed via genetic transformation. Specifically, *E. coli* cells transformed with a plasmid containing *Taq* DNA polymerase and a His tag were cultured at 37 °C, harvested by centrifugation to separate the cells from the culture medium, and resuspended in a buffer comprising 20 mM Tris, pH 8, 500 mM NaCl, and 0.1% Triton X-100. The resuspended cells were incubated on ice and subjected to sonication to disrupt the cell membrane and release proteins. The resulting sample was centrifuged at 13,000 rpm to separate precipitated material from the supernatant, and only the supernatant fraction was collected. To further remove impurities, the collected supernatant was treated at 80°C for 60 minutes, followed by centrifugation to remove precipitated materials.

For anion-exchange column purification, the heat-treated product was first filtered through a 0.2 $\mu$m syringe filter, and then purified using an FPLC system (ÄKTA pure, Cytiva) equipped with an anion-exchange column (HiTrap Q, Cytiva). The anion-exchange column purification was performed by eluting proteins with a 20 mM phosphate buffer while gradually increasing the KCl concentration. Fractions collected from the FPLC were analyzed by SDS-PAGE, and fractions containing a high concentration of the target protein were selected.

To further purify, a His taq purification column (HisTrap from Cytiva) using nickel among metal exchange columns was installed on an FPLC (AKTA pure from Cytiva) equipment. Since the enzyme was designed to include a His tag, the target protein was bound to the column, impurities were washed out using a 20 mM phosphate buffer, and the desired enzyme was eluted as specific fractions by increasing the imidazole concentration up to 500 mM. As described above, each fraction was analyzed by SDS-PAGE, and fractions in which the desired protein was eluted were selected. The purified solution was filtered through a 0.2 $\mu$m syringe filter, and then further concentrated using an Amicon Ultra centrifugal filter unit (30 kDa cut-off). Through this process, proteins larger than the filter unit (including the target enzyme) were concentrated, while smaller proteins below the target protein size were additionally removed.

**Example 3: Preparation of Hot-Start Polymerase**

To prepare a hot-start *Taq* DNA polymerase, the purified *Taq* DNA polymerase was subjected to a hot-start treatment. Specifically, a formaldehyde stock solution (37%, Sigma-Aldrich) was diluted with sterilized distilled water at a volume ratio of 100:168, and the resulting diluted formaldehyde solution was slowly reacted with the enzyme at 37 °C. The final concentration of formaldehyde introduced into the reaction mixture and the reaction time were varied depending on the intended hot-start polymerase product. Specifically, the formaldehyde treatment was performed at final concentrations ranging from 0.0125% to 0.5%, and/or the reaction time was adjusted from 30 minutes to 16 hours, thereby preparing hot-start polymerases. After completion of the reaction, the hot-start polymerase was incubated at low temperature, and the reaction was terminated by adding a solution comprising 20 mM Tris and dithiothreitol (DTT). The final product was concentrated using an Amicon Ultra centrifugal filter unit (30 kDa cut-off) to adjust the protein concentration to a desired level.

## Example 4: Analysis of Activity Profile of Hot-Start Polymerase

The finally concentrated hot-start polymerase was preferably concentrated to a concentration of at least 0.5 mg/mL. For uniformly controlled heat treatment under all conditions, the prepared hot start polymerase was diluted to a concentration of 500 ng/$\mu$L, and 20 $\mu$L aliquots were dispensed into PCR tubes to allow rapid temperature ramp-up and ramp-down. The tubes were placed in a heat block or PCR instrument maintained at 95 °C, and subjected to heat treatment (heat activation) for 0 minutes, 15 minutes, 30 minutes, and 60 minutes, respectively. Immediately after completion of the heat treatment, the samples were immediately cooled by adding 80 ul of ice-cold 1x ThermoPol reaction buffer to quickly lower the temperature to prevent additional heatinduced reaction, and was further diluted to an appropriate concentration level for analysis (50 ng/ul to 6.25 ng/ul). In the case of samples where it is difficult to measure the concentration, the same process as above is followed, but the reaction is started by initially replacing the buffer without more than 1/4 of the thermal stabilizer.

The solution prepared by dilution above was analyzed for the unit of enzyme by fluorescence value for each temperature sample using the method of Example 1. The analyzed samples were plotted with heat treatment time on the x-axis and enzyme specific activity (units per unit mass of enzyme) or unit value (units per unit volume) on the y-axis, and the resulting activity profile is shown in FIG. 4. This provides a unique profile for each hot start polymerase, which can be closely used to determine homology between enzymes in each batch.

## Example 5: Analysis of Activity Profile of Hot-Start KlenTaq Polymerase

Using the same methods as described in Examples 1 to 4, KlenTaq polymerase, which is a mutant of *Taq* DNA polymerase lacking the exonuclease activity domain, was expressed and purified. A hot-start reaction was then applied in the same manner to prepare a hot-start KlenTaq polymerase. It was confirmed that activity profile analysis could be performed for the hot-start KlenTaq polymerase in the same manner as for the hot-start *Taq* DNA polymerase (FIG. 5).

Through this analysis, it was demonstrated that changes in the intrinsic properties of the polymerase result in distinct activity profile shapes and patterns, even when subjected to the same hot-start treatment conditions, and that such differences may ultimately lead to different PCR performance outcomes.

## Example 6: Analysis of Activity Profile of a Hot-Start Polymerase with an Unknown Concentration

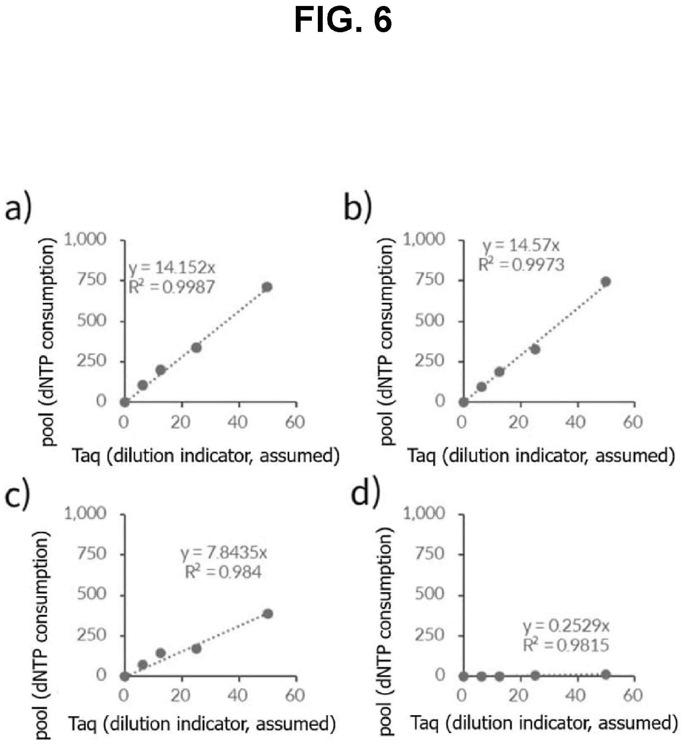

In general, to stably store enzymes over a long period of time, glycerol or saccharides are commonly used to ensure thermal stability. However, such stabilizers make it difficult or impossible to accurately determine the enzyme concentration, and further cause problems during the heat treatment required for hot-start profile (activity profile of a hot-start polymerase) analysis. Accordingly, when analyzing AmpliTaq Gold (Applied Biosystems), which is an external standard or reference enzyme with an unknown concentration, the enzyme was first diluted 1/4-fold with ThermoPol reaction buffer (New England Biolabs), which does not affect thermal stability. After heat treatment (heat activation), the enzyme was serially diluted, and the analysis described in Example 4 was performed. The results are shown in FIG. 6. Specifically, FIG. 6a) shows the result obtained after heat treatment at 95 °C for 60 minutes, FIG. 6 b) shows the result after 30 minutes at 95 °C, FIG. 6 c) shows the result after 15 minutes at 95 °C, and FIG. 6 d) shows the result after 0 minutes at 95 °C.

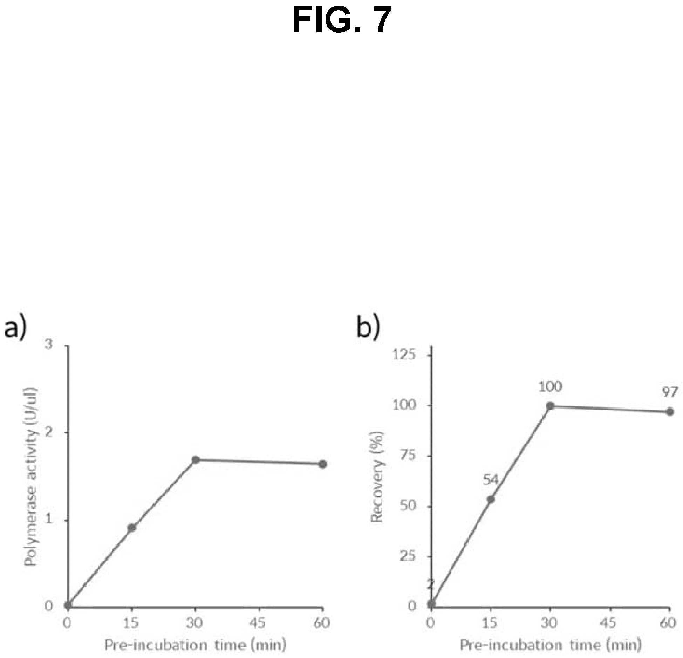

For analytical convenience, the initial enzyme concentration was assumed to be 500 ng/$\mu$L during dilution, and the values indicated on the x-axis of FIG. 6 represent the concentrations of the enzyme diluted from the assumed enzyme concentration. Since the analysis value derived in this way does not know the concentration of the enzyme, it is impossible to infer the specific activity value based on the concentration. However, if the assumed enzyme concentration value of 500 ng/ul is multiplied by the finally calculated assumed specific activity value, it is possible to calculate the unit per unit volume, and thus the shape of the hot start profile could be confirmed through the result (FIG. 7).

In general, the hot-start inhibitory effect is most generally disappeared after heat treatment at 95°C for 30 minutes, and the polymerase activity tends to be strongly expressed under this condition. Therefore, when this condition was substituted as representing 100% recovery of activity, the resulting trend could be graphically illustrated, as shown in FIG. 7 b).

**Example 7: Regression Analysis of the Activity Profile of Hot-Start Polymerase with Ct-Based Units**

In actual diagnostic reagent products, when determining the unit of an enzyme, it is important to maintain consistent quality between a previously used enzyme and a newly produced enzyme by obtaining the same Ct value for the same target. In general, Ct values vary depending on the characteristics of the probe. In particular, probes such as TaqMan probes are significantly affected not only by the polymerization activity of the enzyme but also by its exonuclease activity. Therefore, an additional correction factor is required to match Ct-based unit values with the enzyme unit values analyzed according to Examples 1 and 4.

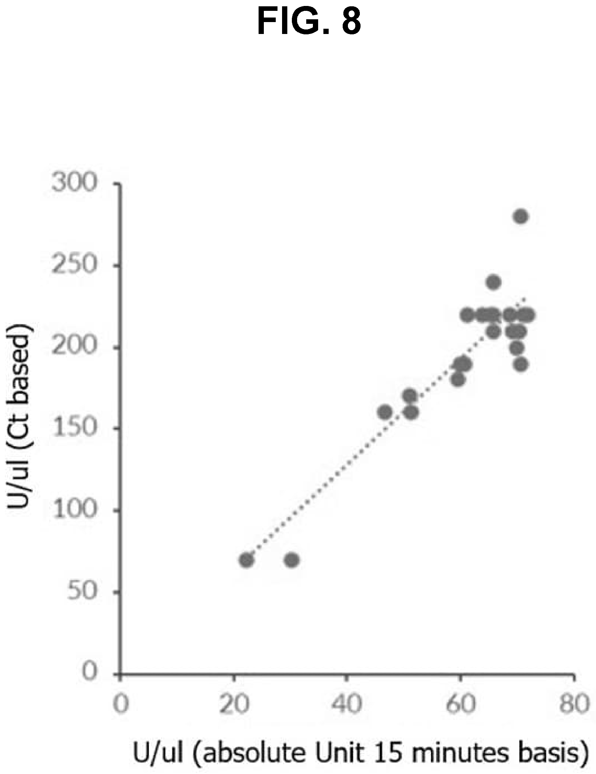

In order to determine the correlation of which factors between the enzyme unit values analyzed according to Examples 1 and 4 and the actual Ct-based unit values, a total of 22 *Taq* DNA polymerase samples produced from different batches were analyzed by regression analysis, in which the respective values were plotted on the x-axis and y-axis (FIG. 8).

The evaluation of Ct-based units was performed using Allplex™ CT/NG/MG/TV assay (Seegene Inc.). The PCR protocol consisted of an initial heat treatment at 95°C for 15 minutes, followed by 45 cycles of 95°C for 3 seconds, 60°C for 10 seconds, and 72°C for 10 seconds, from which Ct values were obtained. Using a previously used enzyme with a predefined unit value (Pre-lot) as a reference, Ct values were first obtained by serially diluting the template DNA. Subsequently, for newly produced DNA polymerases, experiments were repeatedly performed while varying the dilution factor of the polymerase until the same Ct value as that obtained from the Pre-lot was reproduced. Based on this procedure, the dilution factor of the enzyme yielding the same Ct value was determined, and the unit value of the enzyme stock solution was defined to be identical to that of the Pre-lot.

That is, the Ct-based unit measurement was carried out as a method of relatively assuming the unit value of a polymerase capable of reproducing the same Ct value as a reference polymerase, in diagnostic reagent systems using TaqMan probes or similar probe-based detection methods, which rely on the exonuclease activity of the polymerase.

As a result of the regression analysis, it was confirmed that, among the hot-start activity profile values, the activity value at 15 minutes of heat activation showed a statistically significant correlation with the reproduction of identical Ct values for hot-start polymerases subjected to the same hot-start treatment conditions. The coefficient of determination ($R^2$) was 0.9907, and the P-value was in the range of $10^{-23}$, indicating a statistically significant correlation. Accordingly, it was demonstrated that the enzyme activity unit analyzed based on the hot-start activity profile can be meaningfully used to evaluate Ct-based product performance.

Meanwhile, regression analysis between the Ct-based unit values and the activity profile values at 0 minutes, 30 minutes, and 60 minutes of heat activation showed low correlation.

**Example 8: Method of Blending Two or More Polymerases to Mimic an Activity Profile of a Target Hot-Start Polymerase to be Produced**

The values of a hot-start profile (activity profile of a hot-start polymerase) at 0 minutes, 15 minutes, 30 minutes, and 60 minutes can be interpreted as corresponding to, respectively, stability below the target temperature of hot-start, correlation with Ct-based unit measurement, stability over PCR cycling, and longterm storage stability. By taking these factors into consideration, an intended hot-start activity profile for a target polymerase can be designed.

However, as demonstrated in the case of KlenTaq, each polymerase may exhibit variations in its hot-start profile depending on the type of enzyme, changes in the production process, or changes in the hot-start treatment method. Accordingly, it may not always be possible to directly produce a polymerase having an intended hot-start profile. For example, when attempting to produce a polymerase having a hot-start profile similar to that of AmpliTaq Gold, such that the activity level at 15 minutes is maintained at approximately half of the activity level at 30 minutes, this may be achieved by adjusting various experimental conditions. Alternatively, a more convenient approach is to blend two or more polymerases having different hot-start profiles.

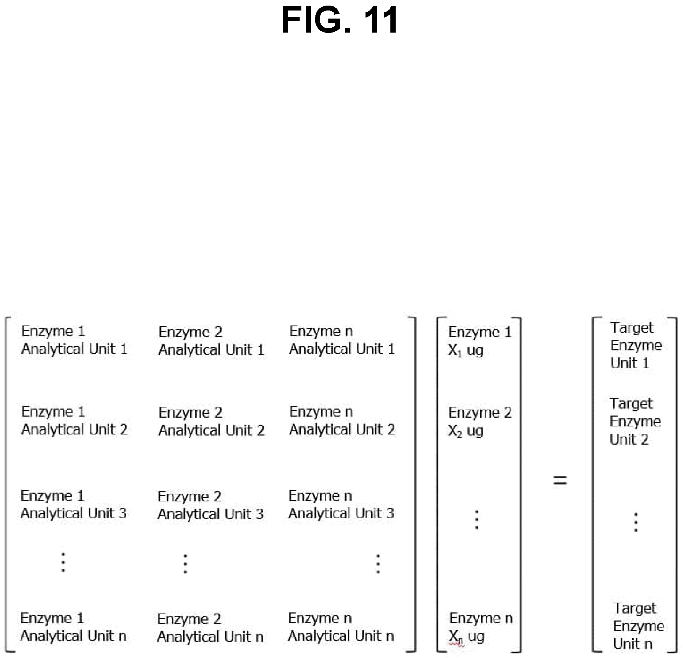

When n polymerases are to be blended, n hot-start activity profiles obtained by heat treatment at 95°C for 0-60 minutes may be used to construct a square matrix, thereby enabling generation of a desired hot-start activity profile regardless of the number of polymerases involved (FIG. 11).

By way of example, assume that polymerase 1 has a hot-start profile with activities of 7.4 U/µg, 10.4 U/µg, and 4.1 U/µg at 15 minutes, 30 minutes, and 60 minutes, respectively, and polymerase 2 has a hot-start profile with activities of 22.1 U/µg, 69.4 U/µg, and 88.6 U/µg at the same respective time points. By solving a linear algebraic equation formed by combining these two profiles, the concentration of each polymerase used can be determined (FIG. 12).

Assuming that two polymerases are blended, AmpliTaq Gold was selected as the target hot-start polymerase. Based on its usage amount of 2 µL per PCR reaction, the activity per reaction was analyzed to be 0.4 U/rxn at 0 minutes, 10.8 U/rxn at 15 minutes, 20.0 U/rxn at 30 minutes, and 19.43 U/rxn at 60 minutes. To mimic this performance, the linear algebraic equation shown in FIG. 12 a) was used to calculate the required amounts ($\mu$g) of polymerase 1 and polymerase 2, which have different hot-start profiles.

As a result, it was determined that blending 1.08 $\mu$g of polymerase 1 with 0.13 $\mu$g of polymerase 2 per reaction reproduces the desired hot-start profile, specifically an activity of 10.8 U/rxn at 15 minutes and 20.0 U/rxn at 30 minutes (FIG. 12 c)).

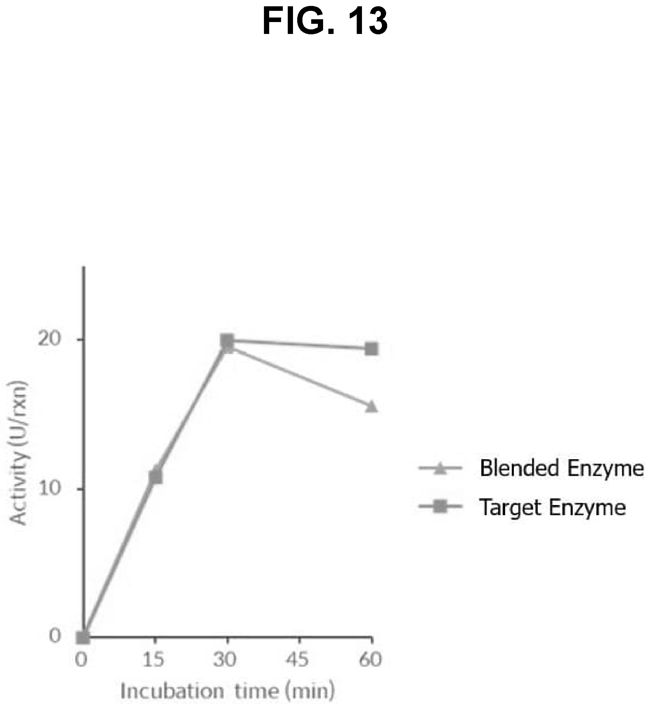

When a blended polymerase was prepared according to the calculated amounts and its hot-start profile was analyzed again, it was confirmed that a hot-start profile very close to the predicted profile was obtained, as shown in FIG. 13.

Having described a preferred embodiment of the present invention, it is to be understood that variants and modifications thereof falling within the spirit of the invention may become apparent to those skilled in this art, and the scope of this invention is to be determined by appended claims and their equivalents.

**Claims**

1.    A method for evaluating a performance of a hot start nucleic acid polymerase, comprising:

   (a) obtaining a product containing a hot start nucleic acid polymerase;
   (b) aliquoting the product containing a hot start nucleic acid polymerase and performing heat activation on each of the aliquoted products for different times;
   (c) measuring an activity of each hot start nucleic acid polymerase contained in each of the heat-activated products for different times; wherein the activity is measured from an intensity of a signal measured by adding each of the heat-activated products for different times to a composition for activity assay of a nucleic acid polymerase and performing nucleic acid polymerization reaction, the composition for activity assay of a nucleic acid polymerase comprises a primer, the primer is extended by the hot start nucleic acid polymerase contained in each of the heat-activated products under polymerization condition to form an extended duplex, and a detectable signal is provided in a manner dependent on the extended duplex, and
   (d) obtaining the activity of the hot start nucleic acid polymerase for the different heat activation times as an activity profile of the hot start nucleic acid polymerase; wherein the activity profile of the hot start nucleic acid polymerase indicates the performance of the hot start nucleic acid polymerase.

2.    A method for obtaining a QC-able hot start nucleic acid polymerase, comprising:

   (a) obtaining a product containing a hot start nucleic acid polymerase;
   (b) aliquoting the product containing a hot start nucleic acid polymerase and performing heat activation on each of the aliquoted products for different times;
   (c) measuring an activity of each hot start nucleic acid polymerase contained in each of the heat-activated products for different times; wherein the activity is measured from an intensity of a signal measured by adding each of the heat-activated products for different times to a composition for activity assay of a nucleic acid polymerase and performing nucleic acid polymerization reaction, the composition for activity assay of a nucleic acid polymerase comprises a primer, the primer is extended by the hot start nucleic acid polymerase contained in each of the heat-activated products under polymerization condition to form an extended duplex, and a detectable signal is provided in a manner dependent on the extended duplex,
   (d) obtaining the activity of the hot start nucleic acid polymerase for the different heat activation times as an activity profile of the hot start nucleic acid polymerase; wherein the activity profile of the hot start nucleic acid polymerase indicates a performance of the hot start nucleic acid polymerase, and
   (e) providing the hot start nucleic acid polymerase that obtains the activity profile.

3.    A method for providing blended hot start nucleic acid polymerases that mimic a performance of a target hot start nucleic acid polymerase, comprising:

   (a) obtaining i) a product containing a target hot start nucleic acid polymerase and ii) products each containing at least two hot start nucleic acid polymerases to be blended;
   (b) i) aliquoting the product containing a target hot start nucleic acid polymerase and performing heat activation on each of the aliquoted products for different times, and ii) aliquoting each of the products each containing the at least two hot start nucleic acid polymerases to be blended and performing heat activation on each of the aliquoted products for different times; wherein the heat activation time of i) is the same as the heat activation time of ii),
   (c) measuring an activity of each target hot start nucleic acid polymerase contained in each of the heat-activated products for different times in i) of step (b), and measuring an activity of each of the at least two hot start nucleic acid polymerases to be blended contained in each of the heat-activated products for different times in ii) of step (b); wherein the activity is measured from an intensity of a signal measured by adding each of the heat-activated products for different times to a composition for activity assay of a nucleic acid polymerase and performing nucleic acid polymerization reaction, the composition for activity assay of a nucleic acid polymerase comprises a primer, the primer is extended by the hot start nucleic acid polymerase contained in each of the heat-activated products under polymerization condition to form an extended duplex, and a detectable signal is provided in a manner dependent on the extended duplex,

(d) obtaining the activity of the target hot start nucleic acid polymerase for the different heat activation times in i) of step (b) as an activity profile of the target hot start nucleic acid polymerase, and obtaining the activity of the at least two hot start nucleic acid polymerases to be blended for the different heat activation times in ii) of step (b) as an activity profile of the at least two hot start nucleic acid polymerases to be blended; wherein the activity profile of the target hot start nucleic acid polymerase indicates a performance of the target hot start nucleic acid polymerase, and each of the activity profiles of the at least two hot start nucleic acid polymerases to be blended indicates a performance of each of the at least two hot start nucleic acid polymerases to be blended,

(e) providing the amount of each of the at least two hot start nucleic acid polymerases to be blended by using the performance of the target hot start nucleic acid polymerase and the performance of the at least two hot start nucleic acid polymerases to be blended to mimic the performance of the target hot start nucleic acid polymerase; and

(f) providing the at least two hot start nucleic acid polymerases blended in the amounts provided in step (e) as the blended hot start nucleic acid polymerases that mimic the performance of the target hot start nucleic acid polymerase.

4. The method according to any one of claims 1 to 3, wherein the hot start nucleic acid polymerase is a chemically modified hot start nucleic acid polymerase, an antibody-based hot start nucleic acid polymerase or an aptamer-based hot start nucleic acid polymerase.

5. The method according to any one of claims 1 to 3, wherein the nucleic acid polymerase is a DNA polymerase.

6. The method according to claim 5, wherein the DNA polymerase is *Taq* DNA polymerase, KlenTaq DNA polymerase, Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase or HIV reverse transcriptase.

7. The method according to any one of claims 1 to 3, wherein the nucleic acid polymerase is prepared by a method comprising the following steps:

(a-1) culturing host cells containing an expression construct for a nucleic acid polymerase; and
(a-2) purifying the nucleic acid polymerase by applying the resultant of step (a-1) to a column chromatography.

8. The method according to any one of claims 1 to 3, wherein the hot start nucleic acid polymerase of step (a) is a nucleic acid polymerase with inhibited polymerization activity.

9. The method according to claim 8, wherein the hot start nucleic acid polymerase is a nucleic acid polymerase whose polymerization activity is suppressed to 1 U/ug or less.

10. The method according to any one of claims 1 to 3, wherein the method further comprises, after step (a), (a-3) diluting the product(s) to a concentration of 0.02 mg/ml to 2 mg/ml.

11. The method according to any one of claims 1 to 3, wherein the heat activation of step (b) is performed on each of the aliquoted products for at least two time intervals among 0 to 60 minutes.

12. The method according to any one of claims 1 to 3, wherein the heat activation of step (b) is performed at 90 to 100°C.

13. The method according to any one of claims 1 to 3, wherein the heat activation of step (b) is performed on each of the aliquoted products at 90 to 100°C for 0 minutes, 15 minutes, 30 minutes, and 60 minutes.

14. The method according to any one of claims 1 to 3, wherein the method further comprises, between steps (b) and (c), (b-1) diluting each of the heat-activated products to a concentration of 6.25 ng/ul to 50 ng/ul.

**15.** The method according to any one of claims 1 to 3, wherein the activity of the hot start nucleic acid polymerase measured in step (c) is provided from a slope measured in a range in which the intensity of a signal increases as a linear function depending on the concentration of the hot start nucleic acid polymerase contained in the product.

**16.** The method according to any one of claims 1 to 3, wherein the activity of the hot start nucleic acid polymerase measured in step (c) is the activity per unit mass of the hot start nucleic acid polymerase or the activity per unit volume of the hot start nucleic acid polymerase.

**17.** The method according to any one of claims 1 to 3, wherein the composition for activity assay of a nucleic acid polymerase further comprises (i) a template, (ii) a label, (iii) dNTP and (iv) a buffer.

**18.** The method according to any one of claims 1 to 3, wherein the primer contained in the composition for activity assay of a nucleic acid polymerase comprises a first primer and a second primer, which are a pair of dimer-forming primers that serve both as a primer and a template; wherein the first primer and the second primer each comprises a 3'-dimer-forming portion, and a nucleotide sequence of the 3'-dimer-forming portion in the first primer is complementary to a nucleotide sequence of the 3'-dimer-forming portion in the second primer.

**19.** The method according to any one of claims 1 to 3, wherein in step (c), the addition of each of the heat-activated products for different times to a composition for activity assay of a nucleic acid polymerase is performed by adding each of the heat-activated products for different times to a composition for activity assay of a nucleic acid polymerase by dilution factor.

**20.** The method according to any one of claims 1 to 3, wherein the nucleic acid polymerization reaction is carried out at 35°C to 75°C for 10 to 60 minutes.

**21.** The method according to claim 17, wherein the label is an intercalating label.

**22.** The method according to claim 21, wherein the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™13 , SYTO™15, SYTO™16, SYTO™20, SYTO™23, TOTO™-3, YOYO™3, GelStar™, thiazole orange, PicoGreen®, EvaGreen® and LAM Fluorescent Dye.

**23.** The method according to any one of claims 1 to 3, wherein the hot start nucleic acid polymerase is hot start *Taq* DNA polymerase or hot start KlenTaq DNA polymerase, and the activity of the hot start nucleic acid polymerase in step (c) is provided as the amount of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming 4.4 ± General formula 1 0.56 nmole of dNTP for 30 minutes at 72°C.

**24.** The method according to any one of claims 1 to 3, wherein the hot start nucleic acid polymerase is hot start *Taq* DNA polymerase or hot start KlenTaq DNA polymerase, and the activity of the hot start nucleic acid polymerase in step (c) is provided as the unit number of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming 4.4 ± General formula 1 0.56 nmole of dNTP for 30 minutes at 72°C.

**25.** The method according to claims 1 or 2, wherein the method further comprises, after step (d), the following steps:

(d-1) obtaining a product containing a hot start nucleic acid polymerase obtained by a different obtaining process from the obtaining process of the product containing a hot start nucleic acid polymerase of step (a);

(d-2) performing steps (b) to (d) using the product obtained in step (d-1) to obtain the activity of the hot start nucleic acid polymerase contained in the product of step (d-1) for the different heat activation times as an activity profile of the hot start nucleic acid polymerase contained in the product of step (d-1); wherein the activity profile of the hot start nucleic acid polymerase contained in the product of step (d-1) indicates the performance of the hot start nucleic acid polymerase contained in the product of step (d-1); and

(d-3) comparing the activity profile of the hot start nucleic acid polymerase contained in the product of step (a) with the activity profile of the hot start nucleic acid polymerase contained in the product in the obtaining process of step (d-1).

26. The method according to claim 3, wherein the step (e) is a step of providing the amount of each of the at least two hot start nucleic acid polymerases to be blended by substituting the performance of the target hot start nucleic acid polymerase and the performance of at least two hot start nucleic acid polymerases to be blended to mimic the performance of the target hot start nucleic acid polymerase into a linear algebraic equation.

27. The method according to claim 26, wherein the linear algebraic equation is expressed by the following formula 1:

## Formula 1

$$[A]_{n \times n}[X]_{n \times 1} = [B]_{n \times 1}$$

wherein the matrix [A] represents the performance of at least two hot start nucleic acid polymerases to be blended, n of the row element showing the size of the matrix in the matrix [A] represents the order of heat activation times and n of the column element in the matrix [A] represents hot start nucleic acid polymerases to be blended; the matrix [X] represents the amount of at least two hot start nucleic acid polymerases to be blended, and n of the row element showing the size of the matrix in the matrix [X] represents hot start nucleic acid polymerases to be blended; and the matrix [B] represents the performance of a target hot start nucleic acid polymerase, and n of the row element showing the size of the matrix in the matrix [B] represents the order of heat activation times.

28. The method according to claim 27, wherein the formula 1 is expressed by the following formula 2:

## Formula 2

$$\begin{bmatrix} a_{11} & a_{12} & \cdots & a_{1n} \\ a_{21} & a_{22} & \cdots & a_{2n} \\ \vdots & \vdots & \vdots & \vdots \\ a_{n1} & a_{n2} & \cdots & a_{nn} \end{bmatrix} \begin{bmatrix} x_1 \\ x_2 \\ \vdots \\ x_n \end{bmatrix} = \begin{bmatrix} b_1 \\ b_2 \\ \vdots \\ b_n \end{bmatrix}$$

wherein the matrix element *a* represents the activity of at least two hot start nucleic acid polymerases to be blended, the row element *n* in the matrix element *a* represents the order of heat activation times, the column element *n* in the matrix element *a* represents hot start nucleic acid polymerases to be blended; the matrix element *x* represents the amount of at least two hot start nucleic acid polymerases to be blended, the row element *n* in the matrix element *x* represents hot start nucleic acid polymerases to be blended; and the matrix element *b* represents the activity of a target hot start nucleic acid polymerase, and the row element *n* in the matrix element *b* represents the order of heat activation times.

# FIG. 1

*100*

*110* — obtaining a product containing a hot start nucleic acid polymerase

*120* — aliquoting the product containing a hot start nucleic acid polymerase and performing heat activation on each of the aliquoted products for different times

*130* — measuring an activity of each hot start nucleic acid polymerase contained in each of the heat-activated products for different times

*140* — obtaining the activity of the hot start nucleic acid polymerase for the different heat activation times as an activity profile of the hot start nucleic acid polymerase

# FIG. 2

# FIG. 3

# FIG. 4

# FIG. 5

# FIG. 6

# FIG. 7

# FIG. 8

# FIG. 9

## 200

210 — obtaining a product containing a hot start nucleic acid polymerase

220 — aliquoting the product containing a hot start nucleic acid polymerase and performing heat activation on each of the aliquoted products for different times

230 — measuring an activity of each hot start nucleic acid polymerase contained in each of the heat-activated products for different times

240 — obtaining the activity of the hot start nucleic acid polymerase for the different heat activation times as an activity profile of the hot start nucleic acid polymerase

250 — providing the hot start nucleic acid polymerase that obtains the activity profile

# FIG. 10

*300*

310 — | obtaining i) a product containing a target hot start nucleic acid polymerase and ii) products each containing at least two hot start nucleic acid polymerases to be blended

320 — | i) aliquoting the product containing a target hot start nucleic acid polymerase and performing heat activation on each of the aliquoted products for different times, and ii) aliquoting each of the products each containing the at least two hot start nucleic acid polymerases to be blended and performing heat activation on each of the aliquoted products for different times

330 — | measuring an activity of each target hot start nucleic acid polymerase contained in each of the heat-activated products for different times in i), and measuring an activity of each of the at least two hot start nucleic acid polymerases to be blended contained in each of the heat-activated products for different times in ii)

340 — | obtaining the activity of the target hot start nucleic acid polymerase for the different heat activation times in i) as an activity profile of the target hot start nucleic acid polymerase, and obtaining the activity of the at least two hot start nucleic acid polymerases to be blended for the different heat activation times in ii) as an activity profile of the at least two hot start nucleic acid polymerases to be blended

350 — | providing the amount of each of the at least two hot start nucleic acid polymerases to be blended by using the performance of the target hot start nucleic acid polymerase and the performance of the at least two hot start nucleic acid polymerases to be blended to mimic the performance of the target hot start nucleic acid polymerase

360 — | providing the at least two hot start nucleic acid polymerases blended in the amounts provided as the blended hot start nucleic acid polymerases that mimic the performance of the target hot start nucleic acid polymerase

# FIG. 11

$$\begin{bmatrix} \begin{array}{ccc} \text{Enzyme 1} & \text{Enzyme 2} & \text{Enzyme n} \\ \text{Analytical Unit 1} & \text{Analytical Unit 1} & \text{Analytical Unit 1} \\\\ \text{Enzyme 1} & \text{Enzyme 2} & \text{Enzyme n} \\ \text{Analytical Unit 2} & \text{Analytical Unit 2} & \text{Analytical Unit 2} \\\\ \text{Enzyme 1} & \text{Enzyme 2} & \text{Enzyme n} \\ \text{Analytical Unit 3} & \text{Analytical Unit 3} & \text{Analytical Unit 3} \\ \vdots & \vdots & \vdots \\\\ \text{Enzyme 1} & \text{Enzyme 2} & \text{Enzyme n} \\ \text{Analytical Unit n} & \text{Analytical Unit n} & \text{Analytical Unit n} \end{array} \end{bmatrix} \begin{bmatrix} \begin{array}{c} \text{Enzyme 1} \\ X_1 \text{ ug} \\\\ \text{Enzyme 2} \\ X_2 \text{ ug} \\\\ \vdots \\\\ \text{Enzyme n} \\ X_n \text{ ug} \end{array} \end{bmatrix} = \begin{bmatrix} \begin{array}{c} \text{Target} \\ \text{Enzyme} \\ \text{Unit 1} \\\\ \text{Target} \\ \text{Enzyme} \\ \text{Unit 2} \\\\ \vdots \\\\ \text{Target} \\ \text{Enzyme} \\ \text{Unit n} \end{array} \end{bmatrix}$$

# FIG. 12

a)

$$\begin{bmatrix} 7.4\ U/\mu g & 22.1\ U/\mu g \\ 10.4\ U/\mu g & 69.4\ U/\mu g \end{bmatrix} \times \begin{bmatrix} \text{Enz.1}\ \mu g/rxn \\ \text{Enz.2}\ \mu g/rxn \end{bmatrix} = \begin{bmatrix} 10.8\ U/rxn \\ 20.0\ U/rxn \end{bmatrix}$$

b)

Enz.1 = 1.08 μg/rxn, Enz.2 = 0.13 μg/rxn

c)

# FIG. 13

# EP 4 786 605 A1

## INTERNATIONAL SEARCH REPORT

<table>
<tr><td colspan="2">International application No.</td></tr>
<tr><td colspan="2"><strong>PCT/KR2024/014643</strong></td></tr>
</table>

### A. CLASSIFICATION OF SUBJECT MATTER

**C12Q 1/48**(2006.01)i; **C12Q 1/686**(2018.01)i

According to International Patent Classification (IPC) or to both national classification and IPC

### B. FIELDS SEARCHED

Minimum documentation searched (classification system followed by classification symbols)

C12Q 1/48(2006.01); C12N 15/09(2006.01); C12P 19/34(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 핫 스타트(hot start), 성능(performance), 모사(mimic), 블렌딩(blending), 열 활성화 (heat activation), 활성 프로파일(activity profile)

### C. DOCUMENTS CONSIDERED TO BE RELEVANT

| Category* | Citation of document, with indication, where appropriate, of the relevant passages | Relevant to claim No. |
|---|---|---|
| A | KR 10-2013-0138700 A (SAMSUNG TECHWIN CO., LTD.) 19 December 2013 (2013-12-19)<br>See claim 10. | 1-28 |
| A | EP 2366802 A1 (AFFYMETRIX, INC.) 21 September 2011 (2011-09-21)<br>See claims 1-6. | 1-28 |
| A | JP 2015-536653 A (MOLECULAR DETECTION ISRAEL LTD.) 24 December 2015 (2015-12-24)<br>See claim 1. | 1-28 |
| A | JP 2013-507910 A (SYNTEZZA MOLECULAR DETECTION ISRAEL LTD.) 07 March 2013 (2013-03-07)<br>See claim 1. | 1-28 |
| A | KR 10-2013-0021351 A (SAMSUNG TECHWIN CO., LTD.) 05 March 2013 (2013-03-05)<br>See claims 1-50. | 1-28 |

☐ Further documents are listed in the continuation of Box C.   ☑ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **13 January 2025** | **14 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

39

## INTERNATIONAL SEARCH REPORT
### Information on patent family members

International application No.

**PCT/KR2024/014643**

| Patent document cited in search report | | | Publication date (day/month/year) | Patent family member(s) | | | Publication date (day/month/year) |
|---|---|---|---|---|---|---|---|
| KR | 10-2013-0138700 | A | 19 December 2013 | US | 2013-0330734 | A1 | 12 December 2013 |
| | | | | US | 9637780 | B2 | 02 May 2017 |
| EP | 2366802 | A1 | 21 September 2011 | EP | 1778866 | A2 | 02 May 2007 |
| | | | | EP | 1778866 | A4 | 03 June 2009 |
| | | | | EP | 1778866 | B1 | 05 October 2011 |
| | | | | JP | 2008-516584 | A | 22 May 2008 |
| | | | | JP | 5020074 | B2 | 05 September 2012 |
| | | | | US | 2006-0029952 | A1 | 09 February 2006 |
| | | | | US | 2007-0031449 | A1 | 08 February 2007 |
| | | | | US | 2008-0138878 | A1 | 12 June 2008 |
| | | | | US | 2011-0189736 | A1 | 04 August 2011 |
| | | | | US | 7628995 | B2 | 08 December 2009 |
| | | | | US | 7700281 | B2 | 20 April 2010 |
| | | | | US | 7951534 | B2 | 31 May 2011 |
| | | | | US | 8404443 | B2 | 26 March 2013 |
| | | | | WO | 2006-005074 | A2 | 12 January 2006 |
| | | | | WO | 2006-005074 | A3 | 19 June 2008 |
| JP | 2015-536653 | A | 24 December 2015 | CN | 105121656 | A | 02 December 2015 |
| | | | | EP | 2920326 | A1 | 23 September 2015 |
| | | | | US | 2015-0275276 | A1 | 01 October 2015 |
| | | | | WO | 2014-076706 | A1 | 22 May 2014 |
| JP | 2013-507910 | A | 07 March 2013 | EP | 2488664 | A2 | 22 August 2012 |
| | | | | EP | 2488664 | A4 | 09 April 2014 |
| | | | | US | 2011-0086354 | A1 | 14 April 2011 |
| | | | | WO | 2011-045744 | A2 | 21 April 2011 |
| | | | | WO | 2011-045744 | A3 | 03 January 2013 |
| KR | 10-2013-0021351 | A | 05 March 2013 | CN | 102918147 | A | 06 February 2013 |
| | | | | CN | 102918147 | B | 12 April 2017 |
| | | | | CN | 106947749 | A | 14 July 2017 |
| | | | | EP | 2576774 | A2 | 10 April 2013 |
| | | | | EP | 2576774 | A4 | 11 December 2013 |
| | | | | EP | 2576774 | B1 | 06 January 2016 |
| | | | | JP | 2013-528384 | A | 11 July 2013 |
| | | | | JP | 2016-146846 | A | 18 August 2016 |
| | | | | JP | 6008846 | B2 | 19 October 2016 |
| | | | | KR | 10-1865870 | B1 | 11 June 2018 |
| | | | | US | 2011-0294674 | A1 | 01 December 2011 |
| | | | | US | 2014-0087968 | A1 | 27 March 2014 |
| | | | | US | 8618253 | B2 | 31 December 2013 |
| | | | | US | 9598729 | B2 | 21 March 2017 |
| | | | | WO | 2011-149255 | A2 | 01 December 2011 |
| | | | | WO | 2011-149255 | A3 | 18 May 2012 |
| | | | | WO | 2011-149255 | A9 | 16 August 2012 |
| | | | | WO | 2011-149255 | A9 | 19 January 2012 |
| | | | | WO | 2011-149255 | A9 | 21 June 2012 |

Form PCT/ISA/210 (patent family annex) (July 2022)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- KR 20230129456
- US 5677152 A
- WO 2019045532 A

## Non-patent literature cited in the description

- **CHIEN A.** ; **EDGAR D. B.** ; **TRELA J. M.** *J. Bacteriol*, 1976, vol. 127, 1550-1557
- **CHARLES C. RICHARDSON** ; **CARL L. SCHILDK-RAUT** ; **H. VASKEN APOSHIAN** ; **ARTHUR KORN-BERG**. *J. Biol. Chem.*, 1964, vol. 239 (1), 222-232
- **MARK A. GRIEP**. *Anal. Biochem.*, 1995, vol. 232, 180-189
- **SCHEIT**. Nucleotide Analogs. John Wiley, 1980
- **UHLMANN** ; **PEYMAN**. *Chemical Reviews*, 1990, vol. 90, 543-584
- **SAMBROOK et al.** Molecular Cloning: A Laboratory Manual. Cold Spring Harbor Laboratory Press, 2001
- **YANOFSKY, C**. *J. Bacteriol.*, 1984, vol. 158, 1018-1024
- **HERSKOWITZ, I** ; **HAGEN, D.** *Annu. Rev. Genet.*, 1980, vol. 14, 399-445
- *Appl. Environ. Microbiol*, 1998, vol. 64, 3932-3938
- *Mol. Gen. Genet*, 1996, vol. 250, 734-741
- **SCHEK, N. et al.** *Mol. Cell Biol*, 1992, vol. 12, 5386-5393
- **KLASENS, B. I. F. et al.** *Nucleic Acids Res.*, 1998, vol. 26, 1870-1876
- **GIL, A. et al.** *Cell*, 1987, vol. 49, 399-406
- **COLE, C. N.** ; **STACY, T. P**. *Mol. Cell Biol*, 1985, vol. 5, 2104-2113
- **BATT, D. B.** ; **CARMICHAEL, G. G**. *Mol. Cell Biol.*, 1995, vol. 15, 4783-4790
- **CAPECCHI, M. R**. *Cell*, 1980, vol. 22, 479
- **HARLAND** ; **WEINTRAUB**. *J. Cell Biol.*, 1985, vol. 101, 1094-1099
- **GRAHAM, F. L. et al.** *Virology*, 1973, vol. 52, 456
- **CHEN** ; **OKAYAMA**. *Mol. Cell Biol.*, 1987, vol. 7, 2745-2752
- **NEUMANN, E. et al.** *EMBO J*, 1982, vol. 1, 841
- **TUR-KASPA et al.** *Mol. Cell Biol.*, 1986, vol. 6, 716-718
- **WONG, T. K. et al.** *Gene*, 1980, vol. 10, 87
- **NICOLAU** ; **SENE**. *Biochim. Biophys. Acta*, 1982, vol. 721, 185-190
- **NICOLAU et al.** *Methods Enzymol.*, 1987, vol. 149, 157-176
- **GOPAL**. *Mol. Cell Biol*, 1985, vol. 5, 1188-1190
- **YANG et al.** *Proc. Natl. Acad. Sci.*, 1990, vol. 87, 9568-9572
- **SAMBROOK et al.** Molecular Cloning, A Laboratory Manual. Cold Spring Harbor Laboratory Press, 2001
- **JOSEPH SAMBROOK et al.** Molecular Cloning: A Laboratory Manual. Cold Spring Harbor Laboratory Press, 2001
- **M. L. M. ANDERSON**. Nucleic Acid Hybridization. Springer-Verlag New York Inc, 1999
- **JESSE L. MONTGOMERY et al.** *Analytical Biochemistry*, 2013, vol. 441 (2), 133-139